(19) Europäisches Patentamt
European Patent Office
Office européen des brevets

(11) Veröffentlichungsnummer: **0 034 760**
**B1**

(12) **EUROPÄISCHE PATENTSCHRIFT**

(45) Veröffentlichungstag der Patentschrift: **30.05.84**

(51) Int. Cl.³: **C 07 D 501/24,** A 61 K 31/545, C 07 D 501/36, C 07 D 501/46

(21) Anmeldenummer: **81100960.4**

(22) Anmeldetag: **11.02.81**

(54) **Cephalosporinderivate, sie enthaltende pharmazeutische Präparate und Verfahren zu ihrer Herstellung.**

(30) Priorität: **23.02.80 DE 3006888**

(43) Veröffentlichungstag der Anmeldung: **02.09.81 Patentblatt 81/35**

(45) Bekanntmachung des Hinweises auf die Patenterteilung: **30.05.84 Patentblatt 84/22**

(84) Benannte Vertragsstaaten: **AT BE CH DE FR GB IT LI NL SE**

(56) Entgegenhaltungen:
**EP - A - 0 002 765**
**FR - A - 2 294 690**
**GB - A - 2 034 692**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Blumbach, Jürgen, Dr., Merziger weg 1a, D-6000 Frankfurt am Main 71 (DE)**
Erfinder: **Dürckheimer, Walter, Dr., Im Lerchenfeld 45, D-6234 Hattersheim am Main (DE)**
Erfinder: **Seeger, Karl, Dr., Schwalbenweg 9, D-6238 Hofheim am Taunus (DE)**

EP 0 034 760 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es sind Cephalosporin-Derivate bekannt, die sich von den erfindungsgemässen Verbindungen dadurch unterscheiden, dass sie in 3-Stellung des Cephem-Rings eine Acetoxymethylgruppe tragen (EP-A-2765) oder dass sie in 5-Stellung des Thiazolylrestes kein Halogenatom besitzen (EP-A-2605, EP-A-5830, FR-A-22 94 690). Gegenüber diesen Verbindungen zeichnen sich die erfindungsgemässen Produkte durch eine unerwartete Überlegenheit ihrer antibakteriellen Wirksamkeit aus. Sie besitzen damit wesentliche therapeutische Vorteile gegenüber den bekannten Verbindungen.

Gegenstand der Erfindung sind Cephalosporinderivate der allgemeinen Formel I

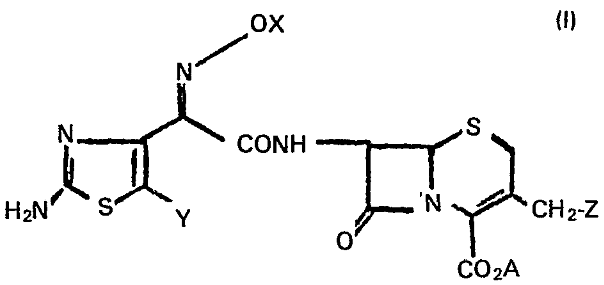

(I)

in der X für Wasserstoff, für $(C_1-C_4)$-Alkyl, das durch Halogen, Hydroxy, Amino, Mono-$(C_1-C_4)$-alkylamino oder Di-$(C_1-C_4)$alkylamino substituiert sein kann, für Carboxymethyl, das auch in Form seiner physiologisch unbedenklichen Salze vorliegen kann, für Alkoxycarbonylmethyl mit 1 bis 4 C-Atomen in Alkoxyteil, für Aminocarbonylmethyl oder Cyanomethyl, wobei die Carboxy-, Alkoxycarbonyl-, Aminocarbonyl- oder Cyanomethylgruppen an der Methylengruppe ein- oder zweimal durch $(C_1-C_4)$-Alkyl substituiert sein können und wobei zwei Alkylsubstituenten auch zu einem 3- bis 6-gliedrigen carbocyclischen Ring geschlossen sein können, Y für Halogen, Z für Azid, für Pyridinium, für 4-Aminocarbonylpyridinium oder für -S-Het steht, wobei Het einen 5-gliedrigen heterocyclischen Ring darstellt, der ausser einem Stickstoffatom als weitere Heteroatome auch noch Stickstoff, Schwefel oder Sauerstoff enthalten kann oder einen 6-gliedrigen heterocyclischen Ring mit 1 bis 3 Stickstoffatomen bedeutet, wobei diese 5- und 6-gliedrigen Ringe auch ganz oder teilweise hydriert und auch noch ein- oder zweifach substituiert sein können durch $(C_1-C_4)$-Alkyl, Trifluormethyl, Carboxy, Carboxymethyl, $(C_1-C_4)$-Alkoxycarbonylmethyl, Hydroxy, Hydroxymethyl, Oxo, Amino, $(C_1-C_4)$-Alkylamino, Di-$(C_1-C_4)$alkylamino, $(C_1-C_4)$-Acylamino, Thienyl oder Pyridyl und A für Wasserstoff, einen leicht abspaltbaren Esterrest oder ein physiologisch unbedenkliches Kation steht.

Steht X für Alkyl mit 1 bis 4 C-Atomen, so seien insbesondere die Reste Methyl, Äthyl, Propyl, iso-Propyl, Butyl und iso-Butyl genannt, die auch substituiert sein können, beispielsweise durch Halogen, insbesondere Brom oder Chlor, durch Hydroxy oder durch Amino, das auch beispielsweise durch Alkyl mit 1 bis 4 C-Atomen, vorzugsweise Methyl, ein- oder zweifach substituiert sein kann.

Steht X für Alkoxycarbonylmethyl mit 1 bis 4 C-Atomen im Alkylteil, so kommen insbesondere Methoxycarbonylmethyl und Äthoxycarbonylmethyl in Betracht.

X in der vorstehenden Bedeutung von Carboxymethyl, das auch in Form seiner physiologisch unbedenklichen Salze vorliegen kann, Alkoxycarbonylmethyl, Aminocarbonylmethyl und Cyanomethyl kann in der Methylengruppe ein- oder zweimal substituiert sein, beispielsweise durch Alkyl mit 1 bis 4 C-Atomen, vorzugsweise Methyl, wobei insbesondere zwei Alkylsubstituenten auch zu einem 3- bis 6-gliedrigen carbocyclischen Ring geschlossen sein können.

Im einzelnen seien als bevorzugte Reste X genannt Wasserstoff, Äthyl, n-Propyl, iso-Propyl, n-Butyl, iso-Butyl, Carboxymethyl, Methoxycarbonylmethyl, Äthoxycarbonylmethyl, Aminocarbonylmethyl und Cyanomethyl. Besonders bevorzugt ist Methyl.

Für Y sind bevorzugte Bedeutungen Brom, Chlor, besonders bevorzugt jedoch Chlor.

Z kann stehen für Azid, Pyridinium, 4-Aminocarbonylpyridinium sowie -S-Het, wobei Het für einen gegebenenfalls ganz oder teilweise hydrierten 5-gliedrigen Heterocyclus, der neben einem Stickstoffatom noch weitere Heteroatome, wie Stickstoff, Schwefel oder Sauerstoff enthalten kann, steht, wie z.B. Thiazol, Thiadiazol, Triazol, Oxadiazol oder Tetrazol, oder für einen gegebenenfalls auch ganz oder teilweise hydrierten 6-gliedrigen Heterocyclus mit 1-3 Stickstoffatomen, wie z.B. Pyridin, Pyrimidin oder Triazin, insbesondere 1,2,4-Triazin-3-yl, der gegebenenfalls ein- oder zweifach substituiert sein kann durch Alkylgruppen mit 1-4 C-Atomen, vorzugsweise Methyl und Äthyl, Trifluormethyl, Carboxy, Carboxymethyl, Alkoxycarbonylmethyl mit 1-4 C-Atomen im Alkylteil, vorzugsweise Methoxy- oder Äthoxycarbonylmethyl, Hydroxy, Hydroxymethyl, Oxo, Amino, Alkyl- und Dialkylamino mit 1-4 C-Atomen im Alkylteil, vorzugsweise Methyl oder Äthyl, Acylamino mit 1-4 C-Atomen im Acylteil, vorzugsweise Formyl oder Acetyl oder durch 5- oder 6-gliedriges Heteroaryl, insbesondere Thienyl und Pyridyl.

Als besonders bevorzugte Bedeutungen von Het seien genannt 1,3,4-Thiadiazol-2-yl, 2-Methyl--1,3,4-thiadiazol-5-yl, 2-Äthyl-1,3,4-thiadiazol-5--yl, 2-Trifluormethyl-1,3,4-thiadiazol-5-yl, 2-Hydroxy-methyl-1,3,4-thiadiazol-5-yl, 2-Amino-1,3,4--thiadiazol-5-yl, 2-Methyl-1,3,4-oxadiazol-5-yl, 2--Hydroxymethyl-1,3,4-oxadiazol-5-yl, 1,3,4-Triazol-2-yl, 1-Methyl-1,3,4-triazol-2-yl, 1,2-Dimethyl--1,3,4-triazol-5-yl, 1,2,3-Triazol-4-yl, 2-Thien-2-yl--1,3,4-triazol-5-yl, 3-Methyl-1,2,4-thiadiazol-5-yl, 5-Carboxymethyl-4-methyl-thiazol-2-yl, Tetrazol-5--yl, 1-Methyl-tetrazol-5-yl, 1-Oxido-pyridin-2-yl, 3,6-Diamino-pyrimidin-2-yl, 6-Hydroxy-4-methyl--4,5-dihydro-5-oxo-1,2,4-triazin-3-yl, 6-Hydroxy-2--methyl-2,5-dihydro-5-oxo-1,2,4-triazin-3-yl.

Als leicht spaltbare Ester in der Bedeutung von A, die den Charakter einer chemischen Schutzgruppe tragen, seien als bevorzugt erwähnt der tert.-Butylester und Trimethylsilylester, sowie der Benzyl-, Benzhydryl-, Trichloräthyl-, p-Methoxybenzyl- oder p-Nitrobenzylester.

Als physiologisch unbedenkliche Kationen A seien beispielsweise genannt ein Alkali-, insbesondere das Natrium- und Kaliumion, ein Erdalkali-, insbesondere das Calcium- und Magnesiumion und Ammoniumion, vorzugsweise jedoch ein Natriumion, sowie ein gegebenenfalls substituiertes alkyliertes Ammoniumion, wobei der Alkylrest 1-4 C-Atome haben kann, wie vorzugsweise Triäthylammonium, Diäthylammonium, Dimethylammonium oder Morpholinium, Benzylammonium, Procainium, L-Argininium und L-Lysinium. Entsprechende physiologisch unbedenkliche Kationen kommen auch für den Fall in Betracht, dass X in Form eines Salzes der Carboxymethylgruppe vorliegt.

Die XO-Gruppe in den Verbindungen der allgemeinen Formeln I, III, IV und V kann in der syn- wie auch in der anti-Form vorliegen. Besonders bevorzugt ist erfindungsgemäss die syn-Form. Die Bezeichnung syn bzw. anti drückt die räumliche Lage relativ zur Carboxyamidgruppe in den Verbindungen I und IV, zur Carboxylgruppe in den Verbindungen III und zur Alkoxycarbonylgruppe in den Verbindungen V aus, wobei die syn-Position diejenige ist, bei der die XO-Gruppe auf der gleichen Seite der C = N-Doppelbindung steht, wie die Carboxyamid-, Carboxyl- oder Alkoxycarbonylgruppe.

2-Aminothiazole der allgemeinen Formel I, III, IV und V können in jeweils zwei tautomeren Formen auftreten, die im Gleichgewichtszustand nebeneinander vorliegen und durch die folgenden Gleichgewichtsgleichungen dargestellt werden können.

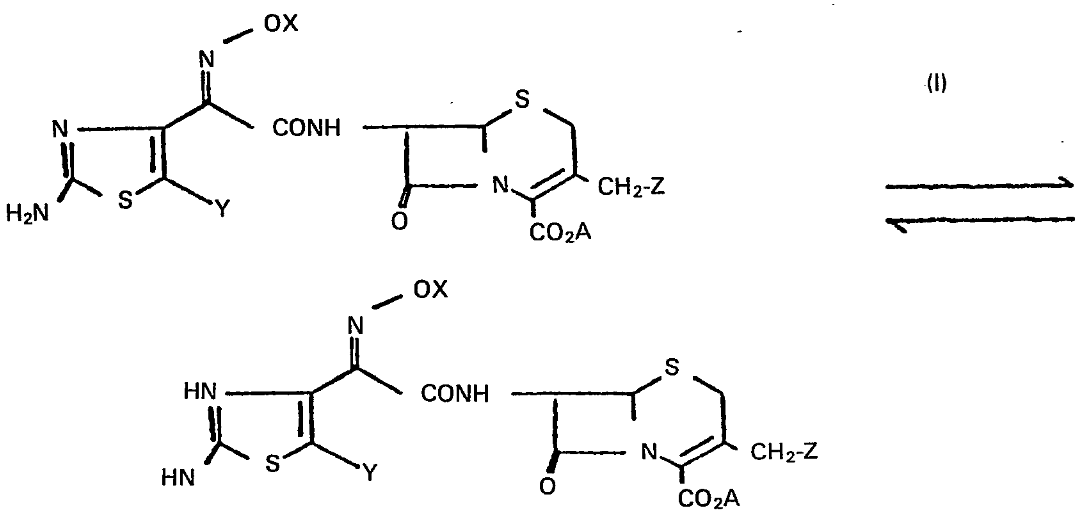

(I)

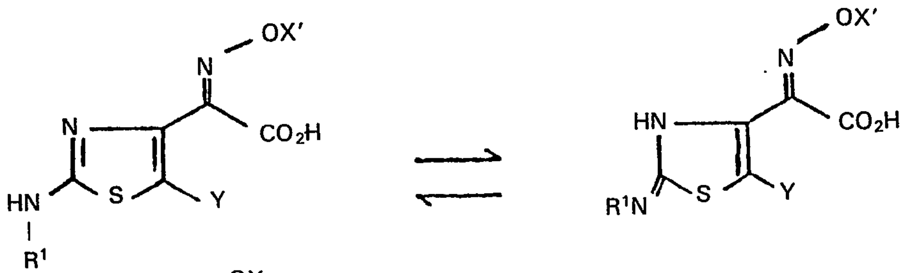

(III)

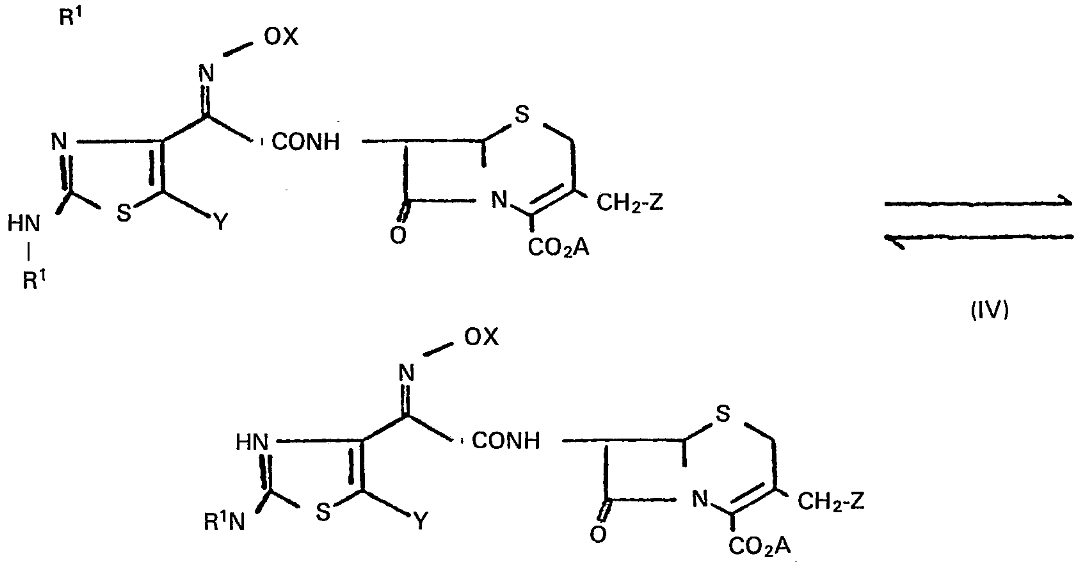

(IV)

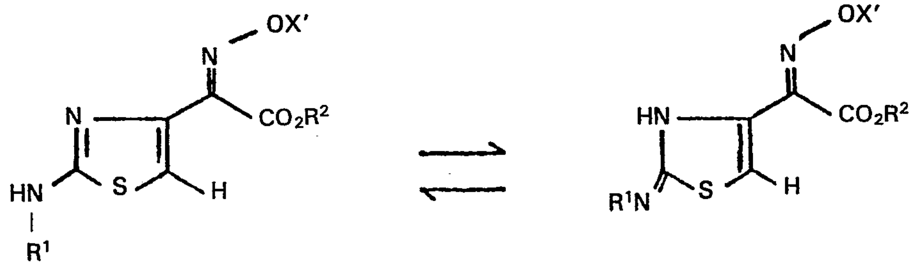

(V)

In der Beschreibung wird davon abgesehen, bei formelmässigen Darstellungen jeweils beide Tautomeren wiederzugeben. Sie beschränkt sich aus Zweckmässigkeitsgründen auf die Wiedergabe des 2-Amino-thiazol-tautomeren

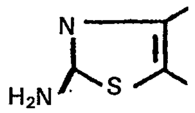

worauf sich auch die Nomenklatur der Verbindungen bezieht.

Gegenstand der Erfindung ist weiterhin ein Verfahren zur Herstellung von Cephemverbindungen der allgemeinen Formel I, das dadurch gekennzeichnet ist, dass man Lactame der allgemeinen Formel II

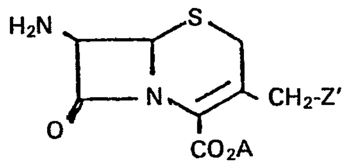

(II)

in der A die oben angegebene Bedeutung hat, und Z′ für Z, Acyloxy mit 1-4 C-Atomen im Acylteil oder Halogen steht, mit einer Carbonsäure der allgemeinen Formel III

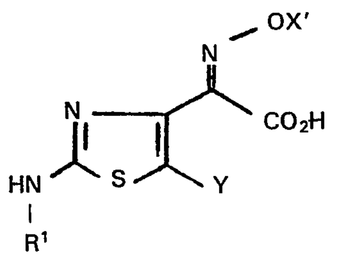

(III)

oder einem aktivierten Derivat derselben, worin Y die oben genannte Bedeutung besitzt, $R^1$ für Wasserstoff oder für eine aus der Peptidchemie bekannte Aminoschutzgruppe und X′ für X, eine leicht abspaltbare Gruppe oder für eine Gruppe

$$-CH_2CO_2R^3$$

steht, in der $R^3$ die Bedeutung eines unter milden Bedingungen abspaltbaren Restes hat, zu einer Verbindung der allgemeinen Formel IV

(IV)

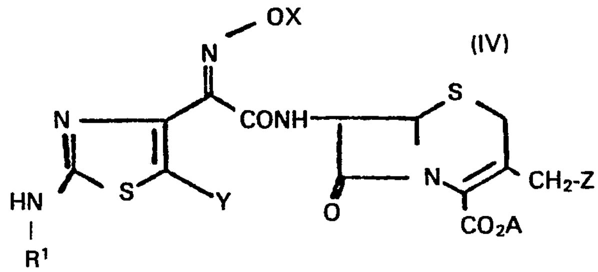

in der $R^1$, X′, Y, Z′ und A die obengenannten Bedeutungen haben, umsetzt, den Rest $R^1$ in der Bedeutung einer Schutzgruppe abspaltet, den Rest X′, soweit er nicht die Bedeutung von X hat, in X überführt und den Rest Z′, soweit er nicht die Bedeutung von Z hat, in Z überführt, wobei die Reihenfolge der Abspaltung von $R^1$ und der Überführung von X′ und Z′ in X und Z beliebig vertauschbar ist.

In den allgemeinen Formeln II, III und IV steht

$R^1$ für Wasserstoff oder für eine aus der Peptidchemie bekannte Amino-Schutzgruppe, wie z.B. gegebenenfalls substituiertes Alkyl, wie vorzugsweise tert.-Butyl, tert.-Amyl, Benzyl, p-Methoxybenzyl, Benzhydryl, Trityl und Phenyläthyl, gegebenenfalls substituiertes aliphatisches Acyl, wie z.B. Formyl, Chloracetyl, Bromacetyl, Trichloracetyl und Trifluoracetyl oder gegebenenfalls substituiertes Alkoxycarbonyl, wie beispielsweise Trichloräthoxycarbonyl oder Benzyloxycarbonyl,

X′ für X, für eine leicht abspaltbare Gruppe wie z.B. Formyl, Trifluoracetyl, Chloracetyl, Bromacetyl, Trityl, tert.-Amyl, tert.-Butyl, Benzhydryl, Tetrahydropyranyl, vorzugsweise jedoch für tert.-Butyl, Trityl und Tetrahydropyranyl, sowie für eine Gruppe der Formel $-CH_2CO_2R^3$, in der $R^3$ die Bedeutung eines unter milden Bedingungen abspaltbaren Restes hat, wie vorzugsweise Trichloräthyl, tert.-Butyl, Benzyl, p-Methoxyphenyl, Benzhydryl oder Trityl,

Z′ für Z, für Acyloxy mit 1-4 C-Atomen im Acylteil, vorzugsweise für Formyloxy und Acetoxy, für Halogen wie vorzugsweise Chlor und Brom.

Als aktivierte Derivate der Carbonsäuren der allgemeinen Formel III eignen sich insbesondere die Halogenide, vorzugsweise Chloride und Bromide, ferner die Anhydride und gemischten Anhydride, die Azide und aktivierten Ester, vorzugsweise die mit p-Nitrophenol, 2,4-Dinitrophenol, Methylencyanhydrin, N-Hydroxysuccinimid und N-Hydroxyphthalimid, besonders bevorzugt mit 1-Hydroxybenzotriazol und 6-Chlor-1-H-hydroxybenzotriazol. Als gemischte Anhydride eignen sich besonders solche mit niederen Alkansäuren, z.B. mit Essigsäure und besonders bevorzugt mit substituierten Essigsäuren, wie z.B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren III, in denen $R^1$ keinen Wasserstoff bedeutet, mit Chlorameisensäurebenzylester, -p-nitrobenzylester, iso-butylester, -äthylester oder -allylester gewinnt.

Die Aktivierung kann auch durch Reaktion der Carbonsäure der allgemeinen Formel III mit einem Reaktionsprodukt aus beispielsweise Phosgen, Thionylchlorid, Thionylbromid, Oxalylchlorid, Phosphorpentachlorid oder Phosphoroxychlorid mit einem N-dialkyl-substituierten Carbonsäureamid, wie z.B. Dimethylformamid, Dimethylacetamid oder N-Methylpyrrolidon erfolgen. Das Reaktionsprodukt aus Dimethylformamid und einem der obengenannten Halogenide wird in der Literatur auch als Vilsmeier-Reagens bezeichnet.

Die aktivierten Derivate können als isolierte Substanzen, aber auch in situ umgesetzt werden. Allgemein erfolgt die Umsetzung der Cephemderivate II mit der Carbonsäure III oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z.B. Diäthyläther, Diisopropyläther und vorzugsweise Tetrahydrofuran und Dioxan; Ketone, wie vorzugsweise Aceton und Butanon; Amide, wie vorzugsweise Dimethylformamid, Dimethylacetamid, N-Methyl-pyrrolidon oder Wasser. Es kann sich auch als vorteilhaft erweisen, Gemische der genannten Lösungsmittel zu verwenden. Dies ist oftmals der Fall, wenn die Cephemverbindung II mit einem in situ erzeugten aktivierten Derivat der Carbonsäure III umgesetzt wird.

Die Umsetzung von Cephemverbindungen II mit Carbonsäuren III bzw. deren aktivierten Derivaten kann in einem Temperaturbereich von etwa —50 bis etwa +80°C, vorzugsweise zwischen —20 und +50°C, besonders bevorzugt jedoch zwischen —20°C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

In einzelnen Fällen kann es sich auch als vorteilhaft erweisen, die freie Carbonsäure der allgemeinen Formel III mit einer Cephemverbindung der allgemeinen Formel II, in der A die Bedeutung eines leicht abspaltbaren Esterrestes, wie vorzugsweise tert.-Butyl oder Trimethylsilyl hat, direkt umzusetzen, wobei man vorteilhaft ein wasserbindendes Mittel in annähernd äquimolarer Menge zusetzt. Als solches kommen zum Beispiel Carbodiimide in Frage, insbesondere Dicyclohexylcarbodiimide. Diese Reaktion wird in inerten Lösungsmitteln, wie vorzugsweise Methylenchlorid, Dimethylformamid, Tetrahydrofuran, Dioxan oder auch Gemischen durchgeführt.

Die Umsetzung von aktivierten Derivaten der Carbonsäuren der allgemeinen Formel III mit Cephemverbindungen der allgemeinen Formel II wird vorzugsweise in alkalischem Milieu oberhalb pH 7 vorgenommen. Man setzt hierfür dem Reaktionsgemisch eine Base zu, wie vorzugsweise Kalium- oder Natriumcarbonat, Kalium- oder Natriumbicarbonat, Kalium- oder Natriumhydroxid, Pyridin oder ein Trialkylamin, wie z.B. Triäthylamin, N-Methylmorpholin, Äthyldiisopropylamin oder Kalium-tert.-butylat.

Die Umsetzung kann vorzugsweise auch so durchgeführt werden, dass ein 1-Hydroxybenzotriazol- oder ein 6-Chlorhydroxybenzotriazolester der Carbonsäuren der allgemeinen Formel III ohne Zusatz eines säurebindenden Mittels in einem Lösungsmittel direkt mit Cephemverbindungen der allgemeinen Formel II umgesetzt werden. Als Lösungsmittel haben sich insbesondere offenkettige und cyclische tertiäre Amide bewährt, besonders bevorzugt Dimethylformamid, Dimethylacetamid und N-Methylpyrrolidon.

Enthalten Cephemverbindungen der allgemeinen Formel IV noch einen Rest $R^1$, X' und/oder Z', so können daraus die Verbindungen der allgemeinen Formel I erhalten werden, indem der Rest $R^1$ — sofern er nicht Wasserstoff bedeutet — abgespalten, X' — sofern er nicht die Bedeutung von X hat — in X überführt und Z' — sofern er nicht für Z steht — in Z umgewandelt wird. Die Reihenfolge der Abspaltung von $R^1$ und der Umwandlungen von X' und Z' in X und Z kann beliebig vertauscht werden, wobei jedoch in der Regel zunächst $R^1$ in Wasserstoff und X' in X umgewandelt und dann erst Z' in Z überführt wird.

Die Überführung des Restes Z' sofern er nicht die Bedeutung von Z hat, in Verbindungen der allgemeinen Formel IV in Z kann durch Umsetzen mit einer den nucleophilen Rest Z enthaltenden Verbindung geschehen. Z' kann dabei stehen für Acyloxy mit 1 bis 4 C-Atomen, insbesondere aber für Formyloxy und Acetoxy, und für Halogen, vorzugsweise Chlor oder Brom. Als Verbindungen, die einen nucleophilen Rest Z enthalten seien insbesondere genannt Stickstoffwasserstoffsäure, insbesondere Pyridin und 4-Aminocarbonylpyridin und Verbindungen der Formel HS-Het, wobei Het die obengenannten Bedeutungen besitzt.

Die Umsetzung wird vorzugsweise so ausgeführt, dass man ein Äquivalent einer Verbindung der allgemeinen Formel IV mit mindestens einem Äquivalent einer einen nucleophilen Rest Z enthaltenden Verbindung insbesondere den vorstehend als vorzugsweise genannten Verbindungen, in einem, die Reaktion nicht behindernden Lösungsmittel umsetzt.

Ein bis zu 10facher Überschuss der nucleophilen Verbindung wirkt sich vorteilhaft auf die Ausbeute aus. Der Überschuss beträgt vorzugsweise 0-200%.

Beispiele für die Reaktion nicht behindernde Lösungsmittel sind Wasser, Aceton, Butanon, Chloroform, Nitrobenzol, Methylenchlorid, Äthylenchlorid, Dimethylformamid, Dimethylacetamid, N-Methylpyrrolidon, Methanol, Äthanol, Essigsäureäthylester, Essigsäurebutylester, Diäthyläther, Diisopropyläther, Tetrahydrofuran und Acetonitril.

Von den Lösungsmitteln können die hydrophilen Lösungsmittel, vorzugsweise Aceton, Methanol, Äthanol, Dimethylformamid und Acetonitril auch im Gemisch mit Wasser verwendet werden.

Die Reaktion wird in einem pH-Bereich von 5 bis 8, vorzugsweise bei neutralem pH ausgeführt.

Wenn die den nucleophilen Rest Z enthaltenden Verbindungen, insbesondere Stickstoffwasserstoffsäure und die Verbindungen der Formel HS-Het, in freier Form verwendet werden, wird die Reaktion

vorzugsweise in Anwesenheit einer Base durchgeführt. Als Base eignet sich beispielsweise eine anorganische Base wie ein Alkalihydroxid, Alkalicarbonat oder Alkalihydrogencarbonat.

Es kann auch eine organische Base wie beispielsweise ein Trialkylamin oder eine quaternäre Ammoniumbase zum Einsatz kommen.

Die den nucleophilen Rest enthaltenden Verbindungen können aber auch in Form ihrer Salze, vorzugsweise ihrer Natrium oder Kalium-Salze eingesetzt werden.

Die Reaktionstemperatur kann in einem weiten Bereich variiert werden. In der Regel wird die Reaktion bei Raumtemperatur oder unter Erwärmen mit Rückflusstemperatur der verwendeten Lösungsmittel oder Lösungsmittelgemische durchgeführt. Zweckmässigerweise werden jedoch 80°C nicht überschritten.

Die Abspaltung von $R^1$ kann mit in der $\beta$-Lactam- und Peptid-Chemie allgemein üblichen schonenden Methoden, wie Hydrolyse in Säuren, vorzugsweise Ameisensäure oder Trifluoressigsäure, oder auch Hydrogenolyse in Gegenwart von Edelmetallkatalysatoren erfolgen. Je nach Schutzgruppe können aber auch spezielle Abspaltungsreagenzien zum Einsatz kommen, wie beispielsweise gegebenenfalls substituierte Thioharnstoffe zum Entfernen von $\alpha$-Halogenacylgruppen.

Auch die Umwandlung der Gruppe X', sofern sie nicht die Bedeutung von X hat, in X kann mit in der $\beta$-Lactam- und Peptid-Chemie üblichen schonenden hydrolytischen oder hydrogenolytischen Methoden herbeigeführt werden, wobei insbesondere Hydrolysen in anorganischen und organischen Säuren, wie vorzugsweise Trifluoressigsäure oder verdünnter Ameisensäure genannt sein sollen.

Bei der erfindungsgemässen Umsetzung anfallende Ester der allgemeinen Formeln I und IV, in denen A die Bedeutung eines leicht abspaltbaren Restes hat, können — falls gewünscht — in literaturbekannter, schonender Weise, z.B. hydrolytisch oder hydrogenolytisch, in Verbindungen der allgemeinen Formel I oder IV umgewandelt werden, in denen A die Bedeutung von Wasserstoff oder einem physiologisch unbedenklichen Kation hat, wie es vorstehend beschrieben worden ist. In analoger Weise kann auch aus dem Rest X' in der Bedeutung von $-CH_2CO_2R^3$ die Gruppe $R^3$ abgespalten werden.

Die zur Acylierung verwendeten Carbonsäuren III lassen sich nach verschiedenen Verfahren herstellen.

So erhält man beispielsweise Verbindungen der allgemeinen Formel III mit Y in der Bedeutung von Halogen, indem Verbindungen der allgemeinen Formel V

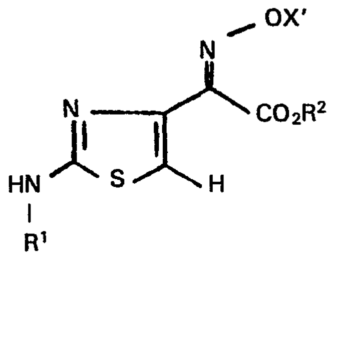

(V).

in der $R^1$ und X' die oben genannten Bedeutungen haben und $R^2$ einen Alkylrest mit 1-4 C-Atomen oder einen Aralkylrest, wie vorzugsweise Benzyl- oder Phenyläthyl, bedeutet, mit einem halogenierenden Reagenz umgesetzt werden und gegebenenfalls der Rest $R^1$ und/oder der Rest X' in die für die folgenden Reaktionen günstigste Form, beispielsweise durch Einführung oder Abspaltung einer Schutzgruppe überführt wird und/oder der so erhaltene Ester V in die Carbonsäure mit der allgemeinen Formel III in an sich bekannter Weise überführt wird.

Als geeignete Halogenierungsmittel können die elementaren Halogene, wie bevorzugt Brom und Chlor, Trihalogen-isocyanursäure, wie vorzugsweise Trichlorisocyanursäure, N-Halogenamide, wie vorzugsweise Chloramin-T, N-Chloracetamid, N-Bromacetamid, N-Halogenimide, wie vorzugsweise N-Chlorsuccinimid, N-Bromsuccinimid, N-Chlor- und N-Bromphthalimid oder Alkylhypochlorite, wie beispielsweise tert.-Butylhypochlorit eingesetzt werden. Die Reaktion wird in der Regel in einem Lösungsmittel durchgeführt, das die Reaktion nicht nachteilig beeinflusst oder sie sogar in die gewünschte Richtung ablaufen lässt. So empfiehlt sich bei Alkylhypochloriten, N-Halogenamiden und N-Halogenimiden ein polares, hydroxylgruppenhaltiges Lösungsmittel, welches die Bildung von positiven Halogenionen fördert, so vorzugsweise Ameisensäure, Eisessig, Wasser, Alkanole wie z.B. Methanol, Äthanol oder Isopropanol. Daneben, vor allem aber bei der Verwendung von elementarem Halogen, können sich auch Lösungsmittel wie Chloroform, Methylenchlorid, Tetrahydrofuran, Dioxan oder Dimethylformamid oder Mischungen derselben untereinander oder mit den oben genannten hydroxylgruppenhaltigen Lösungsmitteln empfehlen.

Die Reaktionstemperatur ist nicht kritisch, sie liegt jedoch bevorzugt im Bereich zwischen etwa —20°C und Raumtemperatur.

Verbindungen der allgemeinen Formel III mit Y in der Bedeutung von Halogen lassen sich weiterhin durch Umsetzung von Thioharnstoff mit Oximen der Formel

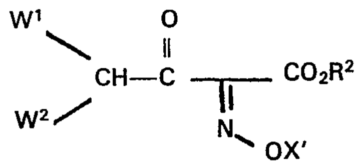

in der $W^1$ und $W^2$ gleich oder verschieden sein können und für Brom oder Chlor stehen, und X' sowie $R^2$ die oben genannten Bedeutungen besitzen, durch gegebenenfalls anschliessende Einführung von $R^1$ in der Bedeutung einer wie oben definierten, aus der Peptid-Chemie bekannten Aminoschutzgruppe und/oder durch Verseifung des so erhaltenen Esters der allgemeinen Formel V zur Carbonsäure der allgemeinen Formel III darstellen.

Zweckmässigerweise erfolgt die Umsetzung mit einer stöchiometrischen Menge Thioharnstoff in einen wasserhaltigen Lösungsmittel, wie z.B. Äthanol oder Aceton. Die Reaktion sollte bei Raumtemperatur durchgeführt werden und maximal etwa 2 bis 3 Stunden dauern.

Die Ausgangsverbindungen der allgemeinen Formel V sind literaturbekannt oder nach literaturbekannten Verfahren herstellbar.

Steht in den allgemeinen Formeln III und V der Rest $R^1$ für eine leicht entfernbare, aus der Peptid-Chemie als Aminoschutzgruppe bekannte Gruppe, so kann ihre Einführung in die Aminogruppe auf die aus der Peptid-Chemie für Aminoschutzgruppen bekannte Weise erfolgen. Stellt $R^1$ beispielsweise die Tritylgruppe dar, so kann ihre Einführung mit Triphenylchlormethan erfolgen, wobei die Reaktion zweckmässigerweise in einem organischen Lösungsmittel, wie beispielsweise halogenierten Kohlenwasserstoffen, in Gegenwart von Basen, wie vorzugsweise Triäthylamin, durchgeführt wird.

Steht in den allgemeinen Formeln III und V X' für eine leicht abspaltbare Gruppe so kann ihre Einführung auf literaturbekannte Weise geschehen, die dem Fachmann für den Schutz von Hydroxylgruppen geläufig ist.

Nicht nur bei der Darstellung der Ausgangsmaterialien III bzw. V, die eine Gruppe

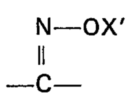

oder

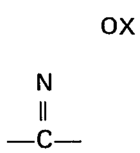

in syn-Position enthalten, sondern auch bei der Darstellung aller Zwischenverbindungen und bei ihrer weiteren Umsetzung zu IV und I ist es zweckmässig, möglichst milde Reaktionsbedingungen, wie sie dem Fachmann für Reaktionen mit syn-Verbindungen aus der Literatur bekannt sind, wie z.B. keine erhöhte Temperatur, keine verlängerten Reaktionszeiten, keine wesentlichen Überschüsse an sauren Reaktionskomponenten usw. anzuwenden, um ein eventuell mögliches Umklappen der Oxim-Gruppe in die anti-Form zu vermeiden.

Die erfindungsgemässen Verbindungen der allgemeinen Formel I zeigen bemerkenswert gute antibakterielle Wirksamkeiten sowohl gegen grampositive als auch gramnegative bakterielle Keime.

Auch gegen penicillinase- und cephalosporinasebildende Bakterien sind die erfindungsgemässen Verbindungen unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemässen Verbindungen charakterisiert sind.

Die erfindungsgemässen Produkte können auch in Kombination mit anderen Wirkstoffen, beispielsweise aus der Reihe der Penicilline, Cephalosporine oder Aminoglykoside zur Anwendung kommen.

Die Verbindungen der allgemeinen Formel I können oral, intramuskulär oder intravenös verabfolgt werden.

Arzneipräparate, die eine oder mehrere Verbindungen der allgemeinen Formel I als Wirkstoff enthalten, können hergestellt werden, indem man die Verbindungen der allgemeinen Formel I mit einem oder mehreren pharmakologisch verträglichen Trägerstoffen oder Verdünnungsmitteln, wie z.B. Füllstoffen, Emulgatoren, Gleitstoffen, Geschmacks-korrigentien, Farbstoffen oder Puffersubstanzen vermischt und in eine geeignete galenische Zubereitungsform bringt, wie beispielsweise Tabletten, Dragees, Kapseln, oder eine zur parenteralen Applikation geeignete Lösung oder Suspension. Als Träger- oder Verdünnungsmittel seien beispielsweise erwähnt Traganth, Milchzucker, Agar-Agar, Polyglykole, Talkum, Äthanol und Wasser. Für die parenterale Applikation kommen vorzugsweise Suspensionen oder Lösungen in Wasser in Betracht. Es ist auch möglich, die Wirkstoffe als solche ohne Träger- oder Verdünnungsmittel in geeigneter Form beispielsweise in Kapseln zu applizieren.

Geeignete Dosen der Verbindungen der allgemeinen Formel I liegen bei etwa 0,4 bis 20 g/Tag, vorzugsweise bei 0,5 bis 4 g/Tag für einen Erwachsenen von etwa 60 kg Körpergewicht. Es können Einzel- oder im allgemeinen Mehrfachdosen verabfolgt werden, wobei die Einzeldosis den Wirkstoff in einer Menge von etwa 50 bis 1000 mg, vorzugsweise 100 bis 500 mg enthalten kann.

Erfindungsgemäss lassen sich ausser den in den Ausführungsbeispielen genannten Verbindungen beispielsweise die in der folgenden Tabelle genannten Verbindungen I darstellen:

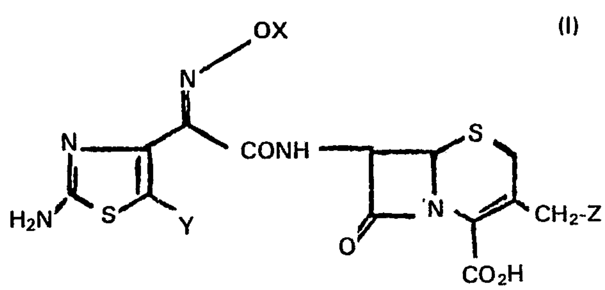

(I)

| X | Y | Z |
|---|---|---|
| H | Cl | 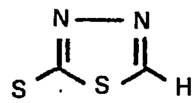 |
| H | Cl | 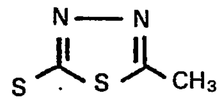 |
| H | Cl | 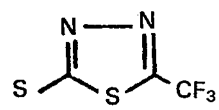 |
| H | Cl | 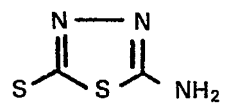 |
| H | Cl | 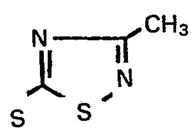 |
| H | Cl | 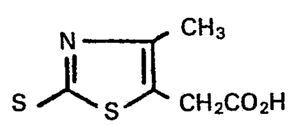 |
| H | Cl | 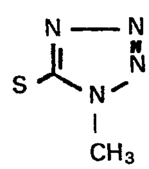 |
| H | Cl | 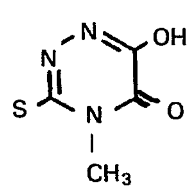 |

Fortsetzung Tabelle

| X | Y | Z |
|---|---|---|
| H | Cl | 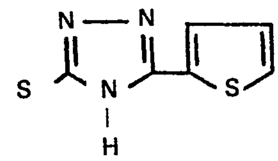 |
| $CH_3$ | Cl | 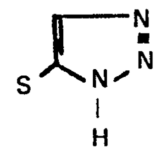 |
| $CH_3$ | Cl | 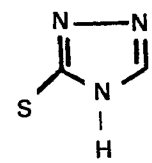 |
| $CH_3$ | Cl | 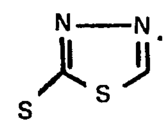 |
| $CH_3$ | Cl | 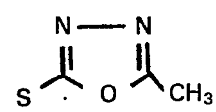 |
| $CH_3$ | Cl | 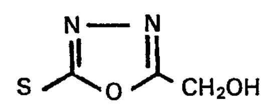 |
| $CH_3$ | Cl | 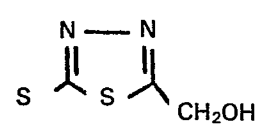 |
| $CH_3$ | Cl | 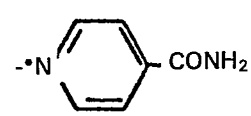 |

Fortsetzung Tabelle

| X | Y | Z |
|---|---|---|
| $CH_3$ | Cl | 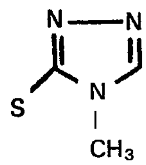 |
| $CH_3$ | Cl | 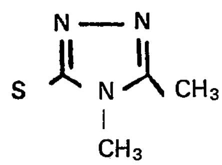 |
| $C_2H_5$ | Cl | 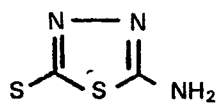 |
| $C_2H_5$ | Cl | 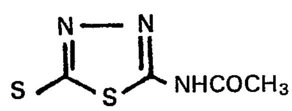 |
| $C_2H_5$ | Cl | 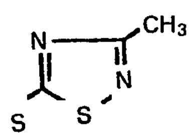 |
| $C_2H_5$ | Cl | 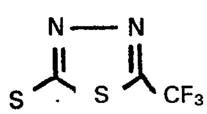 |
| $C_2H_5$ | Cl | 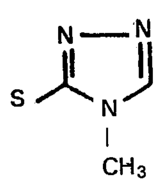 |
| $C_2H_5$ | Cl | 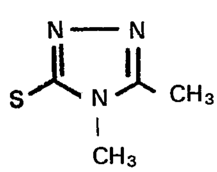 |

Fortsetzung Tabelle

| X | Y | Z |
|---|---|---|
| $C_2H_5$ | Cl | 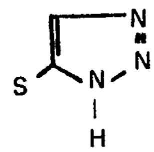 |
| $C_2H_5$ | Cl | 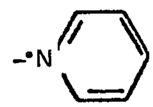 |
| $C_2H_5$ | Cl | 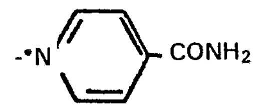 |
| $C_2H_5$ | Cl | 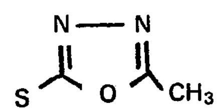 |
| $C_2H_5$ | Cl | 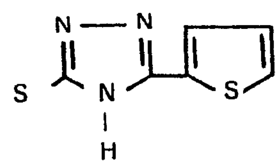 |
| $C_2H_5$ | Cl | 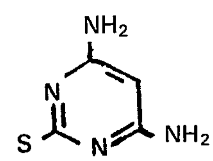 |
| $C_2H_5$ | Cl | 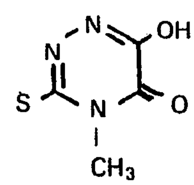 |
| $CH_2CO_2C_2H_5$ | Cl | $N_3$ |

Fortsetzung Tabelle

| X | Y | Z |
|---|---|---|
| $CH_2CO_2C_2H_5$ | Cl | 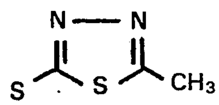 |
| $CH_2CO_2C_2H_5$ | Cl | 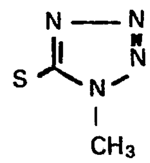 |
| $CH_2CO_2C_2H_5$ | Cl | 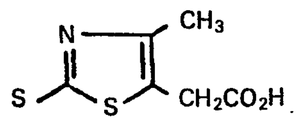 |
| $CH_2CO_2H$ | Cl | $N_3$ |
| $CH_2CO_2H$ | | 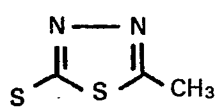 |
| $CH_2CO_2H$ | | 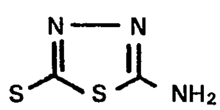 |
| $CH_2CO_2H$ | | 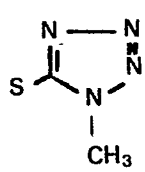 |
| $CH_2CO_2H$ | | 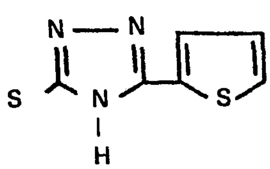 |

Fortsetzung Tabelle

| X | Y | Z |
|---|---|---|
| $CH_3$ | Cl | 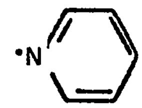 |
| $CH_2CO_2H$ | Cl | 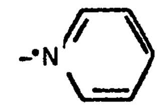 |
| $CH_2CO_2H$ | Cl | 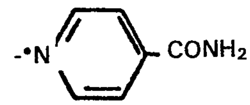 |
| $C(CH_3)_2CO_2H$ | Cl | 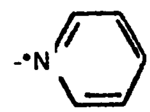 |
| $C(CH_3)_2CO_2H$ | Cl | 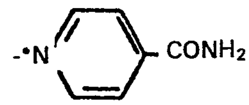 |

Die in den Beispielen angegebenen $R_f$-Werte wurden durch Dünnschichtchromatographie auf Kieselgel-Fertigplatten 60 F 254 der Firma Merck, Darmstadt, ermittelt.

*Beispiel 1*

*7-[α-syn-Methoximino-α-(2-amino-5-chlor-thiazol--4-yl)-acetamido]-3-(1-methyltetrazol-5-yl-thiomethyl)-3-cephem-4-carbonsäure*

940 mg α-syn-Methoximino-α-(2-amino-5-chlorthiazol-4-yl)-essigsäure und 540 mg 1-Hydroxybenzotriazol wurden in 30 ml Tetrahydrofuran gelöst und mit 824 mg Dicyclohexylcarbodiimid versetzt. Nach zweistündigem Rühren bei Raumtemperatur wurde abfiltriert. Zu der Mutterlauge wurde 1,35 g 7-Amino-3-(1-methyltetrazol-5-yl-thiomethyl)-3-cephem-4-carbonsäure, die mit 1,2 g Triäthylamin in 30 ml Methylenchlorid in Lösung gebracht und anschliessend durch Kieselgur filtriert worden war, gegeben und 4 Stunden bei Raumtemperatur gerührt. Die Reaktionslösung wurde durch Kieselgur filtriert und am Rotationsverdampfer eingeengt. Der Rückstand wurde in $H_2O$ bei pH 6,0 aufgenommen, dreimal mit Essigester extrahiert, im Vakuum von Essigesterresten befreit, auf +5°C gekühlt und mit 2N HCl auf pH 1,6 angesäuert. Durch Filtration konnten 1,2 g der Titelverbindung isoliert werden.

NMR (DMSO-$d_6$):
$\delta$ = 9,5 ppm (d, 1H, CONH)
$\delta$ = 7,3 ppm (s, 2H, $NH_2$)
$\delta$ = 5,75 ppm (q, 1H, C-7-H)
$\delta$ = 5,05 ppm (d, 1H, C-6-H)
$\delta$ = 4,3 ppm (breit, $CH_2$-S)
$\delta$ = 3,95 ppm [236 Hz] (s, 3H, Tetr-$CH_3$)
$\delta$ = 3,85 ppm [231 Hz] (s, 3H, $OCH_3$, syn)
$\delta$ = 3,6 ppm (s, breit, C-2-$CH_2$)

*Beispiel 2*

*7-[α-syn-Methoximino-α-(2-amino-5-chlor-thiazol--4-yl)-acetamido]-3-(1-methyltetrazol-5-yl-thiomethyl)-3-cephem-4-carbonsäure*

4,9 g 7-[α-syn-Methoximino-α-(2-amino-5-chlor--thiazol-4-yl)-acetamido]-cephalosporansäure und 1,5 g Natrium-1-methyl-tetrazol-5-yl-thiolat wurden in 100 ml $H_2O$ bei pH 6,5 bis 7,0 und 60°C 3 Stunden gerührt. Nach dem Abkühlen wurde mit 2N HCl auf pH 4,5 gestellt, zweimal mit Essigester extrahiert und die wässrige Phase nach Entfernen von Essigester-Resten im Vakuum bei 5°C mit 2N HCl auf pH 2,0 gestellt. Nach 1/4 stündigem Rühren wurde abfiltriert und getrocknet. Man erhielt so 3,7 g der Titelverbindung in ihren physikalischen Konstanten identisch mit der in Beispiel 1 dargestellten Verbindung.

*Beispiel 3*

*7-[α-syn-Methoximino-α-(2-amino-5-chlor-thiazol--4-yl)-acetamido]-3-azidomethyl-3-cephem-4--carbonsäure*

2,32 g α-syn-Methoximino-α-(2-amino-5-chlor--thiazol-4-yl)-essigsäure wurden in 25 ml Tetrahydrofuran gelöst, mit 1,36 g 1-Hydroxy-benzotriazol versetzt und auf 45°C erwärmt. Nach Zugabe von 2,16 g Dicyclohexylcarbodiimid wurde 2 Stunden bei Raumtemperatur gerührt, vom ausgefallenen Harnstoff (2,18 g) abgesaugt und der Harnstoff mit 15 ml Tetrahydrofuran gewaschen.

Die vereinigten Filtrate wurden mit einer Lösung aus 2,55 g 7-Amino-3-azido-methyl-3-cephem-4--carbonsäure und 3,0 g Triäthylamin in 40 ml $CH_2Cl_2$ vereinigt und 6 Stunden bei Raumtemperatur gerührt. Die gebildeten Kristalle wurden abfiltriert, mit wenig $CH_2Cl_2$ gewaschen. Man erhält so 2,3 g des Triäthylammoniumsalzes der Titelverbindung.

NMR (DMSO-$d_6$):
$\delta$ = 9,5 ppm (d, 1H, CONH)
$\delta$ = 7,4 ppm (breit, 2H, $NH_2$)
$\delta$ = 5,6 ppm (q, 1H, C-7-H)
$\delta$ = 5,1 ppm (d, 1H, C-6-H)
$\delta$ = 3,8 ppm [231 Hz] (s, 3H, $OCH_3$, syn)
$\delta$ = 4,3 ppm (breit, $CH_2$-S)
$\delta$ = 3,4 ppm (breit, C-2-$CH_2$)
$\delta$ = 3,0 ppm (q, 6H, N-$CH_2$)
$\delta$ = 1,2 ppm (t, 9H, $NCH_2CH_3$)

2,0 g des Triäthylammoniumsalzes der Titelverbindung wurden bei 0°C in 50 ml $H_2O$ gelöst und mit 2N HCl auf pH 2,1 gestellt. Durch Filtration konnten 1,36 g der Titelverbindung isoliert werden.

NMR (DMSO-$d_6$):
$\delta$ = 9,5 ppm (d, 1H, CONH)
$\delta$ = 7,3 ppm (breit, 2H, $NH_2$)
$\delta$ = 5,6 ppm (q, 1H, C-7-H)
$\delta$ = 5,05 ppm (d, 1H, C-6-H)
$\delta$ = 4,4 ppm (AB, $CH_2$-$N_3$)
$\delta$ = 3,8 ppm [232 Hz] (s, 3H, $OCH_3$, syn)
$\delta$ = 3,4 ppm (breit, C-2-$CH_2$)

*Beispiel 4*

*7-[α-syn-Methoximino-α-(2-amino-5-chlor-thiazol--4-yl)-acetamido]-3-(2-methyl-1,3,4-thiadiazol-5--yl-thiomethyl)-3-cephem-4-carbonsäure*

1,18 g α-syn-Methoximino-α-(2-amino-5-chlor--thiazol-4-yl)-essigsäure und 680 mg 1-Hydroxy--benzotriazol wurden in 50 ml Tetrahydrofuran gelöst und mit 1,03 g Dicyclohexylcarbodiimid versetzt. Nach zweistündigem Rühren wurde abfiltriert. Die Mutterlauge wurde mit einer Suspension aus 1,72 g 7-Amino-3-(2-methyl-1,3,4-thiadiazol-2-yl--thiomethyl)-3-cephem-4-carbonsäure und 2,0 g Triäthylamin in 50 ml $CH_2Cl_2$ versetzt.

Es wurde über Nacht bei Raumtemperatur gerührt, das Lösungsmittel im Vakuum abdestilliert, in 60 ml $H_2O$ aufgenommen und bei pH 4,5 viermal mit Essigester extrahiert. Nach Abkühlen auf 5°C wurde mit 2N HCl auf pH 2,3 angesäuert und vom Niederschlag abfiltriert. Es konnten so 1,13 g der Titelverbindung isoliert werden.

NMR (DMSO-$d_6$):
$\delta$ = 9,5 ppm (d, 1H, CONH)
$\delta$ = 7,3 ppm (s, 2H, $NH_2$)
$\delta$ = 5,75 ppm (q, 1H, C-7-H)
$\delta$ = 5,05 ppm (d, 1H, C-6-H)
$\delta$ = 4,3 ppm (AB, 2H, $CH_2$-S)
$\delta$ = 3,8 ppm [230 Hz] (s, 3H, $OCH_3$, syn)
$\delta$ = 3,3 ppm (breit, C-2-$CH_2$)
$\delta$ = 2,65 ppm (s, 3H, $CH_3$)

*Beispiel 5*

*7-[α-syn-Methoximino-α-(2-amino-5-chlor-thiazol--4-yl)-acetamido]-3-(2-methyl-1,3,4-thiadiazol-5--yl-thiomethyl)-3-cephem-4-carbonsäure*

4,9 g 7-[2-syn-Methoximino-α-(2-amino-5-chlor--thiazol-4-yl)-acetamido]-cephalosporansäure und 1,5 g 2-Methyl-1,3,4-thiadiazol-5-thiol wurden in 70 ml $H_2O$ durch Zusatz von festem $NaHCO_3$ bei pH 6,5-7,0 in Lösung gebracht.

Nach Zugabe von 30 ml Aceton wurde die Lösung bei pH 6,5-7,0 und Rückfluss 2 1/2 Stunden gerührt. Anschliessend wurde das Aceton am Rotationsverdampfer abgezogen und die Lösung bei 5°C unter Rühren mit 2N HCl auf pH 2,3 angesäuert. Durch Filtration konnten 2,7 g der Titelverbindung isoliert werden, in ihren physikalischen Konstanten identisch mit der in Beispiel 4 dargestellten Verbindung.

*Beispiel 6*

*7-[α-syn-Methoximino-α-(2-amino-5-chlor-thiazol--4-yl)-acetamido]-3-(2-amino-1,3,4-thiadiazol-5--yl-thiomethyl)-3-cephem-4-carbonsäure*

Setzt man analog Beispiel 5 2,5 g 7-[α-syn-Me-

thoximino-α-(2-amino-5-chlor-thiazol-4-yl)-acetamido]-cephalosporansäure mit 0,7 g 2-Amino-1,3,4-thiadiazol-5-thiol um, so erhält man 1,8 g der Titelverbindung.

NMR ($DMSO-d_6$):
δ = 9,5 ppm (d, 1H, CONH)
δ = 7,0-7,5 ppm (breites Signal, 4H, Thiazol-$NH_2$ und Thiadiazol-$NH_2$)
δ = 5,65 ppm (q, 1H, C-7-H)
δ = 5,0 ppm (d, 1H, C-6-H)
δ = 4,1 ppm (AB, $CH_2S$)
δ = 3,9 ppm [231 Hz] (s, 3H, $OCH_3$, syn!)
δ = 3,2 ppm (breit, C-2-$CH_2$)

*Beispiel 7*

*7-[α-Methoximino-α-(2-amino-5-chlor-thiazol-4-yl)-acetamido]-3-(3-methyl-1,2,4-thiadiazol-5-yl-thiomethyl)-3-cephem-4-carbonsäure*

Setzt man analog Beispiel 5 2,5 g 7-[α-syn-Methoximino-α-(2-amino-5-chlor-thiazol-4-yl)-acetamido]-cephalosporansäure mit 0,75 g 3-Methyl-1,2,4-thiadiazol-5-thiol um, so erhält man 1,3 g der Titelverbindung.

NMR ($DMSO-d_6$):
δ = 9,5 ppm (d, 1H, CONH)
δ = 7,45 ppm (s, breit, 2H, $NH_2$)
δ = 5,70 ppm (q, 1H, C-7-H)
δ = 5,05 ppm (d, 1H, C-6-H)
δ = 4,15 ppm (AB, $CH_2S$)
δ = 3,95 ppm [232 Hz] (s, 3H, $OCH_3$, syn!)
δ = 3,1 ppm (breit, C-2-$CH_2$)

*Beispiel 8*

*7-[α-syn-Methoximino-α-(2-amino-5-chlor-thiazol-4-yl)-acetamido]-3-[2-(thien-2-yl)-1-H-1,3,4-triazol-5-yl-thiomethyl]-3-cephem-4-carbonsäure*

2,35 g α-syn-Methoximino-α-(2-amino-5-chlor-thiazol-4-yl)-essigsäure wurden in 25 ml Tetrahydrofuran gelöst, mit 1,35 g 1-Hydroxy-benzotriazol versetzt und auf 45°C erwärmt. Nach Zugabe von 2,16 g Dicyclohexylcarbodiimid wurde 2 Stunden bei Raumtemperatur gerührt und anschliessend vom ausgefallenen Harnstoff (1,9 g) abfiltriert.

Das Filtrat wurde mit einer Lösung aus 4,0 g 7-Amino-3-(2-thien-2-yl-1-H-1,3,4-triazol-2-yl-thiomethyl)-3-cephem-4-carbonsäure und 3,0 g Triäthylamin in 30 ml $CH_2Cl_2$ vereinigt, 8 Stunden bei Raumtemperatur gerührt und anschliessend 1 Woche im Kühlschrank belassen. Das Lösungsmittel wurde im Vakuum entfernt, der Rückstand in 100 ml $H_2O$ aufgenommen und bei pH 4,5 dreimal mit Essigester extrahiert. Die wässrige Phase wurde sodann bei 0-5°C mit 2N HCl auf pH 2,0 angesäuert. Es konnten so 400 mg der Titelverbindung isoliert werden.

NMR ($DMSO-d_6$):
δ = 9,5 ppm (d, 1H, CONH)
δ = 7,4 ppm (breit, $NH_2$)
δ = 7,7-7,5 ppm (m, 2H, thienyl)
δ = 7,5-7,0 ppm (m, 2H, thienyl)
δ = 5,7 ppm (q, 1H, C-7-H)
δ = 5,1 ppm (d, 1H, C-6-H)
δ = 4,3 ppm (breit, 2H, $CH_2$-S)
δ = 3,9 ppm [232 Hz] (s, 3H, $OCH_3$, syn)
δ = 3,7 ppm (breit, C-2-$CH_2$)

*Beispiel 9*

*7-[α-syn-Methoximino-α-(2-amino-5-chlor-thiazol-4-yl)-acetamido]-3-(5-carboxymethyl-4-methyl-thiatol-2-yl-thiomethyl)-3-cephem-4-carbonsäure*

940 mg α-syn-Methoximino-α-(2-amino-5-chlor-thiazol-4-yl)-essigsäure und 540 mg 1-Hydroxybenzotriazol wurden in 40 ml Tetrahydrofuran gelöst und mit 824 mg Dicyclohexylcarbodiimid versetzt. Nach einstündigem Rühren wurde abfiltriert. Zu der Mutterlauge wurde eine Lösung von 2,0 g 7-Amino-3-(5-carboxy-methyl-4-methyl-thiazol-2-yl-thiomethyl)-3-cephem-4-carbonsäure, gelöst in 30 ml Methylenchlorid, 2,0 g Triäthylamin, 2,0 ml Dimethylformamid und 3,0 ml $H_2O$ gegeben. Es wurde über Nacht bei Raumtemperatur gerührt, das Lösungsmittel im Vakuum abdestilliert, mit 50 ml $H_2O$ versetzt und bei pH 4,5 viermal mit Essigester extrahiert. Nach Abkühlen auf +5°C wurde mit 2N HCl auf pH 2,0 angesäuert und der Niederschlag durch Filtration gesammelt: 1,26 g.

NMR ($DMSO-d_6$):
δ = 9,5 ppm (d, 1H, CONH)
δ = 7,4 ppm (s, breit, 2H, $NH_2$)
δ = 5,75 ppm (q, 1H, C-7-H)
δ = 5,05 ppm (d, 1H, C-6-H)
δ = 4,2 ppm (AB, $CH_2$-S)
δ = 3,9 ppm [230 Hz] (s, 3H, $OCH_3$, syn)
δ = 3,8 ppm [224 Hz] (s, 2H, $CH_2$-$CO_2H$)
δ = 3,3 ppm (breit, C-2-$CH_3$)
δ = 2,2 ppm (s, 3H, $CH_3$)

*Beispiel 10*

*7-[α-syn-Methoximino-α-(2-amino-5-chlor-thiazol-4-yl)-acetamido]-3-(2-trifluormethyl-1,3,4-thiadiazol-5-yl-thiomethyl)-3-cephem-4-carbonsäure*

Setzt man analog Beispiel 4 2 g 7-Amino-3-(2-trifluormethyl-1,3,4-thiadiazol-5-yl-thiomethyl)-3-cephem-4-carbonsäure mit dem Reaktionsprodukt aus 1,18 g α-syn-Methoximino-α-(2-amino-5-chlor-thiazol-4-yl)-essigsäure, 680 mg 1-Hydroxy-benzotriazol und 1,03 g Dicyclohexylcarbodiimid um, so erhält man 1,7 g der Titelverbindung.

NMR ($DMSO-d_6$):
δ = 9,5 ppm (d, 1H, CONH)
δ = 7,2 ppm (breit, 2H, $NH_2$)
δ = 5,65 ppm (q, 1H, C-7-H)
δ = 5,0 ppm (d, 1H, C-6-H)
δ = 4,3 ppm (breites s, $CH_2$-S)
δ = 3,85 ppm [231 Hz] (s, 3H, $OCH_3$, syn)
δ = 3,25 ppm (breit, C-2-$CH_2$)

*Beispiel 11*

*7-[α-syn-Methoximino-α-(2-amino-5-chlor-thiazol-4-yl)-acetamido]-3-(2-trifluormethyl-1,3,4-thiadiazol-5-yl-thiomethyl)-3-cephem-4-carbonsäure*

5,0 g 7[α-syn-Methoximino-α-(2-amino-5-chlor-

thiazol-4-yl)-acetamido]-cephalosporansäure und 2,2 g 2-Trifluormethyl-1,3,4-thiadiazol-5-thiol wurden in 70 ml $H_2O$ durch Zusatz von festem $NaHCO_3$ bei pH 6,5 in Lösung gebracht. Nach Zusatz von 40 ml Essigester wurde 2 Stunden zum Rückfluss erhitzt, abgekühlt und die wässrige Phase abgetrennt. Reste von Essigester wurden aus der wässrigen Phase am Rotationsverdampfer entfernt. Bei 5°C wurde unter Rühren mit 2N HCl auf pH 2,0 angesäuert. Durch Filtration konnten 4,3 g der Titelverbindung isoliert werden, in ihren physikalischen Konstanten identisch mit der in Beispiel 10 dargestellten Verbindung.

*Beispiel 12*

*7-[α-syn-Methoximino-α-(2-amino-5-chlor-thiazol-4-yl)-acetamido]-3-(1,2-dimethyl-1,3,4-traizol-5-yl-thiomethyl)-3-cephem-4-carbonsäure*

Setzt man analog Beispiel 11 2,5 g 7-[α-syn-Methoximino-α-(2-amino-5-chlor-thiazol-4-yl)-acetamido]-cephalosporansäure mit 0,7 g 1,2-Dimethyl-1,3,4-triazol-5-thiol um, so erhält man 2,25 g der Titelverbindung.

NMR (DMSO-$d_6$):
δ = 9,5 ppm (d, 1H, CO<u>NH</u>)
δ = 7,15 ppm (s, breit, 2H, $NH_2$)
δ = 5,70 ppm (q, 1H, C-7-H)
δ = 5,05 ppm (d, 1H, C-6-H)
δ = 4,1 ppm (s, breit, $CH_2$-S)
δ = 3,85 ppm [231 Hz] (s, 3H, $OCH_3$, syn)
δ = 3,6 ppm (s, verbreitert, 3H, N-$\underline{CH}_3$ + C-2-$CH_2$)
δ = 2,3 ppm (s, 3H, $CH_3$)

*Beispiel 13*

*7-[α-syn-Methoximino-α-(2-amino-5-chlor-thiazol-4-yl)-acetamido]-3-(6-hydroxy-4-methyl-4,5-dihydro-1,2,4-triazin-5-on-3-yl-thiomethyl)-3-cephem-4-carbonsäure*

Setzt man analog Beispiel 11 2,5 g 7-[α-syn-Methoximino-α-(2-amino-5-chlor-thiazol-4-yl)-acetamido]-cephalosporansäure mit 0,85 g 6-Hydroxy-4-methyl-3-mercapto-4,5-dihydro-1,2,4-triazin-5-on um, so erhält man 1,97 g der Titelverbindung.

NMR (DMSO-$d_6$):
δ = 9,5 ppm (d, 1H, CONH)
δ = 7,2 ppm (s, breit, $NH_2$)
δ = 5,75 ppm (q, 1H, C-7-H)
δ = 5,1 ppm (d, 1H, C-6-H)
δ = 4,05 ppm (s, breit, $CH_2$-S)
δ = 3,8 ppm [230 Hz] (s, 3H, $OCH_3$, syn)
δ = 3,5 ppm (s, breit, C-2-$CH_2$)
δ = 3,3 ppm (s, 3H, N-$CH_3$)

*Beispiel 14*

*7-[α-syn-Methoximino-α-(2-amino-5-chlor-thiazol-4-yl)-acetamido]-3-(4,6-diamino-pyrimidin-2-yl-thiomethyl)-3-cephem-4-carbonsäure*

Setzt man analog Beispiel 11 2,5 g 7-[α-syn-Methoximino-α-(2-amino-5-chlor-thiazol-4-yl)-acetamido]-cephalosporansäure mit 0,8 g 4,6-Diamino-pyrimidin-2-thiol um, so erhält man 1,5 g der Titelverbindung.

NMR (DMSO-$d_6$):
δ = 9,5 ppm (d, 1H, CONH)
δ = 7,15 ppm (s, breit, $NH_2$)
δ = 5,75 ppm (q, 1H, C-7-H)
δ = 5,0-5,1 ppm (m, 2H, C-6-H + Pyrimidin-H)
δ = 4,2 ppm (breit, $CH_2$-S)
δ = 3,85 ppm [231 Hz] (s, 3H, $OCH_3$, syn)
δ = 3,5 ppm (breit, C-2-$CH_2$)

*Beispiel 15*

*7-[α-syn-Äthoximino-α-(2-amino-5-chlor-thiazol-4-yl)-acetamido]-3-(1-methyl-tetrazol-5-yl-thiomethyl)-3-cephem-4-carbonsäure*

Setzt man analog Beispiel 1 990 mg α-syn-Äthoximino-α-(2-amino-5-chlor-thiazol-4-yl)-essigsäure, 540 mg 1-Hydroxy-benzotriazol und 830 mg Dicyclohexylcarbodiimid mit 1,35 g 7-Amino-3-(1-methyl-tetrazol-5-yl-thiomethyl)-3-cephem-4-carbonsäure um, so erhält man 0,95 g der Titelverbindung.

NMR (DMSO-$d_6$):
δ = 9,5 ppm (d, 1H, CON<u>H</u>)
δ = 7,2 ppm (breit, 2H, $NH_2$)
δ = 5,7 ppm (q, 1H, C-7-H)
δ = 5,0 ppm (d, 1H, C-6-H)
δ = 4,15 ppm (q, 2H, O$\underline{CH_2}$$CH_3$)
δ = 4,3 ppm (breit, C$\underline{H_2}$-S)
δ = 4,0 ppm (s, 3H, Tetr.-$CH_3$)
δ = 3,6 ppm (s, breit, C-2-$CH_2$)
δ = 1,25 ppm (t, 3H, $OCH_2CH_3$)

*Beispiel 16*

*7-[α-syn-Äthoximino-α-(2-amino-5-chlor-thiazol-4-yl)-acetamido]-3-(2-Methyl-1,3,4-thiadiazol-5-yl-thiomethyl)-3-cephem-4-carbonsäure*

Setzt man analog Beispiel 4 1,23 g α-syn-Äthoximino-α-(2-amino-5-chlor-thiazol-4-yl)-essigsäure, 680 mg 1-Hydroxybenzotriazol und 1,03 g Dicyclohexylcarbodiimid mit 1,72 g 7-Amino-3-(2-methyl-1,3,4-thiadiazol-2-yl-thiomethyl)-3-cephem-4-carbonsäure um, so erhält man 2,15 g der Titelverbindung.

NMR (DMSO-$d_6$):
δ = 9,5 ppm (d, 1H, CON<u>H</u>)
δ = 7,3 ppm (s, breit, $NH_2$)
δ = 5,75 ppm (q, 1H, C-7-H)
δ = 5,0 ppm (d, 1H, C-6-H)
δ = 4,1 ppm (q, 2H, O$\underline{CH_2}$$CH_3$)
δ = 4,3 ppm (AB, 2H, $CH_2$-S)
δ = 3,2 ppm (breit, C-2-$CH_2$)
δ = 2,65 ppm (s, 3H, $CH_3$)
δ = 1,2 ppm (t, 3H, $OCH_2CH_3$)

*Beispiel 17*

*7-[α-syn-Äthoximino-α-(2-amino-5-chlor-thiazol-4-yl)-acetamido]-3-azidomethyl-3-cephem-4-carbonsäure*

Setzt man analog Beispiel 3 1,23 g α-syn-Äthoximino-α-(2-amino-5-chlor-thiazol-4-yl)-essigsäure,

680 mg 1-Hydroxybenzotriazol und 1,03 g Dicyclohexylcarbodiimid mit 1,25 g 7-Amino-3-azidomethyl-3-cephem-4-carbonsäure um, so erhält man 1,9 g der Titelverbindung.

NMR ($DMSO-d_6$):
δ = 9,5 ppm (d, 1H, CONH)
δ = 7,3 ppm (s, breit, $NH_2$)
δ = 5,65 ppm (q, 1H, C-7-H)
δ = 5,05 ppm (d, 1H, C-6-H)
δ = 4,15 ppm (q, 2H, $OCH_2CH_3$)
δ = 4,4 ppm (AB, $CH_2$-$N_3$)
δ = 3,4 ppm (breit, C-2-$CH_2$)
δ = 1,25 ppm (t, 3H, $OCH_2CH_3$)

*Beispiel 18*

*7-[α-syn-Methoximino-α-(2-amino-5-chlorthiazol-4-yl)-acetamido]-3-(6-hydroxy-4-methyl-4,5-dihydro-1,2,4-triazin-5-on-3-yl-thiomethyl)-3-cephem-4-carbonsäure*

4,7 g α-syn-Methoximino-α-(2-amino-5-chlorthiazol-4-yl)-essigsäure werden in 60 ml Tetrahydrofuran unter Zusatz von 2,7 g 1-Hydroxybenzotriazol in Lösung gebracht und mit 4,12 g Dicyclohexylcarbodiimid versetzt. Nach einstündigem Rühren bei Raumtemperatur wird abfiltriert. Die Mutterlauge wird zu einer Lösung aus 7,4 g 7-Amino-3-(6-hydroxy-4-methyl-4,5-dihydro-1,2,4-triazin-5-on-3-yl-thiomethyl)-3-cephem-4-carbonsäure und 2 g Triäthylamin in 100 ml Tetrahydrofuran, 16 ml Dimethylformamid und 52 ml $H_2O$ gegeben. Nach 4 1/2-stündigem Rühren bei Raumtemperatur wurde am Rotationsverdampfer auf ca. 50 ml eingeengt, auf 0°C gekühlt und mit 2N HCl auf pH 4 eingestellt. Es wurde dreimal mit je 50 ml Essigester extrahiert, die wässrige Phase im Vakuum von Resten Essigester befreit und mit 2N HCl auf pH 2,0 angesäuert. Der entstandene Niederschlag wurde abgesaugt und getrocknet. Man erhielt 3,7 g der Titelverbindung in ihrer physikalischen Konstanten identisch mit der in Beispiel 13 hergestellten Verbindung.

*Beispiel 19*

*7-[α-syn-Methoximino-α-(2-amino-5-chlorthiazol-4-yl)-acetamido]-3-(6-hydroxy-4-äthyl-4,5-dihydro-1,2,4-triazin-5-on-3-yl-thiomethyl)-3-cephem-4-carbonsäure*

Verfährt man analog Beispiel 18 und setzt 7,7 g 7-Amino-3-(6-hydroxy-4-äthyl-4,5-dihydro-1,2,4-triazin-5-on-3-yl-thiomethyl)-3-cephem-4-carbonsäure ein, so erhält man 3,2 g der Titelverbindung.

NMR ($DMSO-d_6$):
δ = 12,4 ppm (s, OH)
δ = 9,5 ppm (d, 1H, $CONH_4$)
δ = 7,1 ppm (s, breit, $NH_2$)
δ = 5,75 ppm (q, 1H, C-7-H)
δ = 5,1 ppm (d, 1H, C-6-H)
δ = 4,05 ppm (breit, $CH_2S$)
δ = 3,8 ppm [230 Hz] (s, 3H, $OCH_3$, syn)
δ = 1,2 ppm (t, 3H, $CH_2CH_3$)

*Beispiel 20*

*7-[α-syn-Methoximino-α-(2-amino-5-chlorthiazol-4-yl)acetamido]-3-(6-hydroxy-2-methyl-2,5-dihydro-1,2,4-triazin-5-on-3-yl-thiomethyl)-3-cephem-4-carbonsäure*

Verfährt man analog Beispiel 18 und setzt 7,4 g 7-Amino-3-(6-hydroxy-2-methyl-2,5-dihydro-1,2,4-triazin-5-on-3-yl-thiomethyl)-3-cephem-4-carbonsäure ein, so erhält man 2,53 g der Titelverbindung.

NMR ($DMSO-d_6$):
δ = 9,5 ppm (d, 1H, CONH)
δ = 7,1 ppm (s, breit, $NH_2$)
δ = 5,75 ppm (dd, 1H, C-7-H)
δ = 5,1 ppm (d, 1H, C-6-H)
δ = 4,2 ppm (AB, $CH_2S$)
δ = 3,8 ppm [231 Hz] (s, 3H, $OCH_3$, syn)
δ = 3,55 ppm (s, 3H, N-$CH_3$)

**Patentansprüche für die Vertragsstaaten:**
BE, CH, DE, FR, GB, IT, LI, NL, SE

1. Cephalosporinderivate der allgemeinen Formel

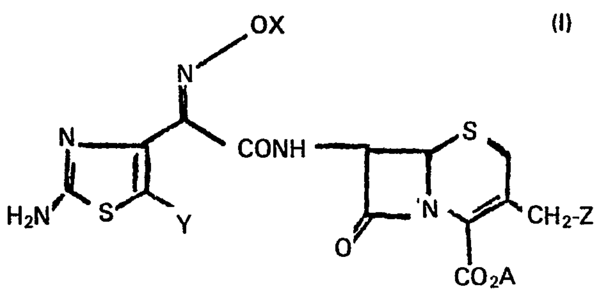

(I)

in der X für Wasserstoff, für ($C_1$-$C_4$)-Alkyl, das durch Halogen, Hydroxy, Amino, Mono-($C_1$-$C_4$)-alkylamino oder Di-($C_1$-$C_4$)alkylamino substituiert sein kann, für Carboxymethyl, das auch in Form seiner physiologisch unbedenklichen Salze vorliegen kann, für Alkoxycarbonylmethyl mit 1 bis 4 C-Atomen im Alkoxyteil, für Aminocarbonylmethyl oder Cyanomethyl, wobei die Carboxy-, Alkoxycarbonyl-, Aminocarbonyl- oder Cyanomethylgruppen an der Methylengruppe ein- oder zweimal durch ($C_1$-$C_4$)-Alkyl substituiert sein können und wobei zwei Alkylsubstituenten auch zu einem 3- bis 6-gliedrigen carbocyclischen Ring geschlossen sein können, Y für Halogen, Z für Azid, für Pyridinium, für 4-Aminocarbonylpyridinium oder für -S-Het steht, wobei Het einen 5-gliedrigen heterocyclischen Ring darstellt, der ausser einem Stickstoffatom als weitere Heteroatome auch noch Stickstoff, Schwefel oder Sauerstoff enthalten kann oder einen 6-gliedrigen heterocyclischen Ring mit 1 bis 3 Stickstoffatomen bedeutet, wobei diese 5- und 6-gliedrigen Ringe auch ganz oder teilweise hydriert und auch noch ein- oder zweifach substituiert sein können durch ($C_1$-$C_4$)-Alkyl, Trifluormethyl, Carboxy, Carboxymethyl, ($C_1$-$C_4$)-Alkoxycarbonylmethyl, Hydroxy, Hydroxymethyl, Oxo, Amino, ($C_1$-$C_4$)-Alkylamino, Di-($C_1$-$C_4$)alkyl-

amino, $(C_1-C_4)$-Acylamino, Thienyl oder Pyridyl und A für Wasserstoff, einen leicht abspaltbaren Esterrest oder ein physiologisch unbedenkliches Kation steht.

2. Verfahren zur Herstellung von Cephalosporinderivaten der allgemeinen Formel I, dadurch gekennzeichnet, dass man Lactame der allgemeinen Formel II

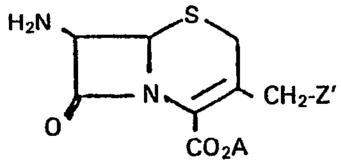
(II)

in der A die obengenannte Bedeutung hat und Z' für Z, Acyloxy mit 1-4 C-Atomen im Acylteil oder Halogen steht, mit einer Carbonsäure der allgemeinen Formel III

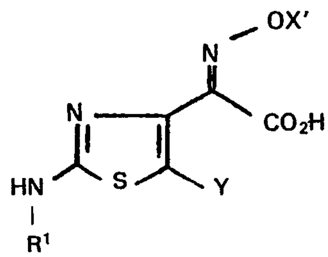
(III)

oder einem aktivierten Derivat derselben, worin Y die obengenannte Bedeutung besitzt, $R^1$ für Wasserstoff oder für eine aus der Peptidchemie bekannte Aminoschutzgruppe und X' für X, eine leicht abspaltbare Gruppe oder für eine Gruppe

$$—CH_2CO_2R^3$$

steht, in der $R^3$ die Bedeutung eines unter milden Bedingungen abspaltbaren Restes hat, zu einer Verbindung der allgemeinen Formel IV

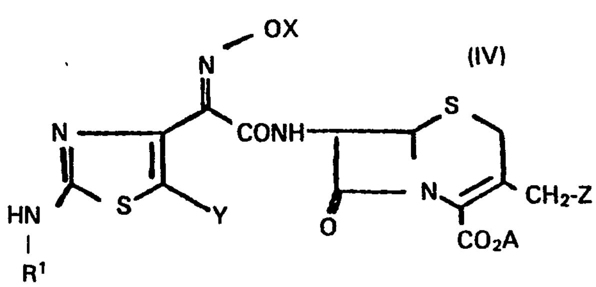
(IV)

in der $R^1$, X', Y, Z' und A die obengenannten Bedeutungen haben, umsetzt, den Rest $R^1$ in der Bedeutung einer Schutzgruppe abspaltet, den Rest X', soweit er nicht die Bedeutung von X hat, in X überführt und den Rest Z', soweit er nicht die Bedeutung von Z hat, in Z überführt, wobei die Reihenfolge der Abspaltung von $R^1$ und der Überführung von X' und Z' und X und Z beliebig vertauschbar ist.

3. Pharmazeutisches Präparat gekennzeichnet durch einen Gehalt an Verbindungen der allgemeinen Formel I.

4. Verfahren zur Herstellung von pharmazeutischen Präparaten, dadurch gekennzeichnet, dass eine Verbindung der allgemeinen Formel I, gegebenenfalls mit pharmazeutisch üblichen Trägerstoffen oder Verdünnungsmitteln, in eine pharmazeutisch geeignete Verabreichungsform gebracht wird.

**Patentanspruch für den Vertragsstaat:**
AT

Verfahren zur Herstellung von Cephalosporinderivaten der allgemeinen Formel I

(I)
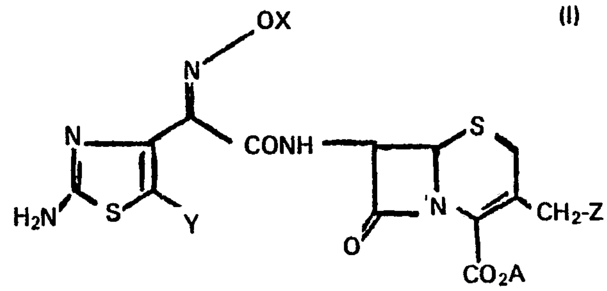

in der X für Wasserstoff, für $(C_1-C_4)$-Alkyl, das durch Halogen, Hydroxy, Amino, Mono-$(C_1-C_4)$-alkylamino oder Di-$(C_1-C_4)$alkylamino substituiert sein kann, für Carboxymethyl, das auch in Form seiner physiologisch unbedenklichen Salze vorliegen kann, für Alkoxycarbonylmethyl mit 1 bis 4 C-Atomen im Alkoxyteil, für Aminocarbonylmethyl oder Cyanomethyl, wobei die Carboxy-, Alkoxycarbonyl-, Aminocarbonyl- oder Cyanomethylgruppen an der Methylengruppe ein- oder zweimal durch $(C_1-C_4)$-Alkyl substituiert sein können und wobei zwei Alkylsubstituenten auch zu einem 3- bis 6-gliedrigen carbocyclischen Ring geschlossen sein können, Y für Halogen, Z für Azid, für Pyridinium, für 4-Aminocarbonylpyridinium oder für -S-Het steht, wobei Het einen 5-gliedrigen heterocyclischen Ring darstellt, der ausser einem Stickstoffatom als weitere Heteroatome auch noch Stickstoff, Schwefel oder Sauerstoff enthalten kann oder einen 6-gliedrigen heterocyclischen Ring mit 1 bis 3 Stickstoffatomen bedeutet, wobei diese 5- und 6-gliedrigen Ringe auch ganz oder teilweise hydriert und auch noch ein- oder zweifach substituiert sein können durch $(C_1-C_4)$-Alkyl, Trifluormethyl, Carboxy, Carboxymethyl, $(C_1-C_4)$-Alkoxycarbonylmethyl, Hydroxy, Hydroxymethyl, Oxo, Amino, $(C_1-C_4)$-Alkylamino, Di-$(C_1-C_4)$alkylamino, $(C_1-C_4)$-Acylamino, Thienyl oder Pyridyl und A für Wasserstoff, einen leicht abspaltbaren Esterrest oder ein physiologisch unbedenkliches Kation steht, dadurch gekennzeichnet, dass man Lactame der allgemeinen Formel II

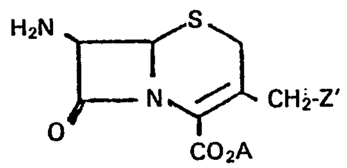
(II)

in der A die obengenannte Bedeutung hat und Z' für Z, Acyloxy mit 1-4 C-Atomen im Acylteil oder Halogen steht, mit einer Carbonsäure der allgemeinen Formel III

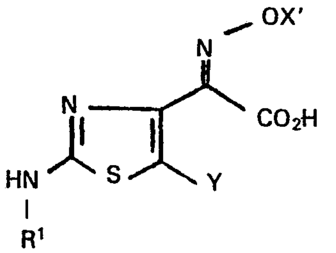

(III)

oder einem aktivierten Derivat derselben, worin Y die obengenannte Bedeutung besitzt, $R^1$ für Wasserstoff oder für eine aus der Peptidchemie bekannte Aminoschutzgruppe und X' für X, eine leicht abspaltbare Gruppe oder für eine Gruppe

$$-CH_2CO_2R^3$$

steht, in der $R^3$ die Bedeutung eines unter milden Bedingungen abspaltbaren Restes hat, zu einer Verbindung der allgemeinen Formel IV

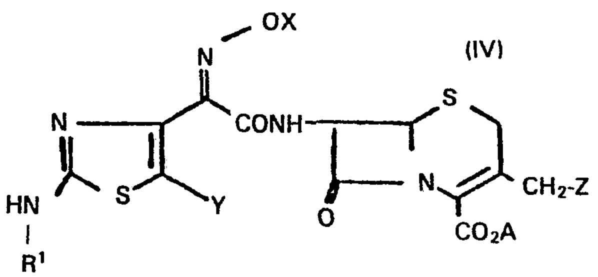

(IV)

in der $R^1$, X', Y, Z' und A die obengenannten Bedeutungen haben, umsetzt, den Rest $R^1$ in der Bedeutung einer Schutzgruppe abspaltet, den Rest X', soweit er nicht die Bedeutung von X hat, in X überführt und den Rest Z', soweit er nicht die Bedeutung von Z hat, in Z überführt, wobei die Reihenfolge der Abspaltung von $R^1$ und der Überführung von X' und Z' in X und Z beliebig vertauschbar ist.

**Revendications pour l'Etats contractants:**
BE, CH, DE, FR, GB, IT, LI, NL, SE

1. Dérivés de céphalosporine de formule générale I

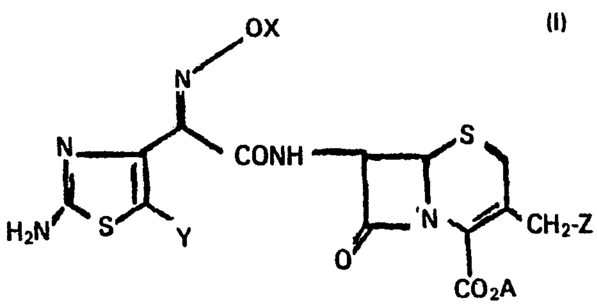

(I)

dans laquelle, X représente l'hydrogène, un groupe alcoyle en $C_1$-$C_4$, qui peut être substitué par un halogène, un groupe hydroxy, amino, mono-alcoyl ($C_1$-$C_4$)-amino ou dialcoyl($C_1$-$C_4$)-amino, un groupe carboxyméthyle qui peut être également présent sous la forme de ses sels physiologiquement acceptables, un groupe alcoxycarbonylméthyle ayant de 1 à 4 atomes de carbone dans la partie alcoxy, un groupe aminocarbonylméthyle ou cyanométhyle, dans lesquels les groupes carboxy, alcoxycarbonyle, aminocarbonyle ou cyanométhyle peuvent être une ou deux fois substitués par un groupe alcoyle en $C_1$-$C_4$ sur le groupe méthylène, et deux substituants alcoyle pouvant également être fermés en un noyau carbocyclique ayant de 3 à 6 chaînons, Y représente un halogène. Z représente un groupe azide, pyridinium, 4-aminocarbonylpyridinium ou un groupe S-Het, dans lequel Het représente un noyau hétérocyclique à 5 chaînons, qui, en dehors d'un atome d'azote, peut encore contenir d'autres hétéroatomes tels que l'azote, le soufre ou l'oxygène, ou un noyau hétérocyclique à 6 chaînons ayant de 1 à 3 atomes d'azote, ces noyaux à 5 et 6 chaînons pouvant être également totalement ou partiellement hydrogénés et substitués également encore une ou deux fois par un groupe alcoyle en $C_1$-$C_4$, trifluorométhyle, carboxy, carboxyméthyle, alcoxy($C_1$-$C_4$)-carbonylméthyle, hydroxy, hydroxyméthyle, oxo, amino, alcoyl($C_1$-$C_4$)--amino, dialcoyl($C_1$-$C_4$)-amino, acyl($C_1$-$C_4$)-amino, thiényle ou pyridyle, et A représente l'hydrogène, un groupe ester facilement éliminable ou un cation physiologiquement compatible.

2. Procédé de préparation de dérivés de céphalosporine de formule générale I, caractérisé en ce qu'on fait réagir des lactames de formule générale II

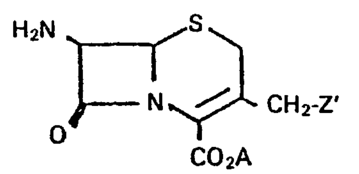

(II)

dans laquelle A a la signification indiquée ci-dessus et Z' représente Z, un groupe acyloxy ayant de 1 à 4 atomes de carbone dans la partie acyle ou un halogène, avec un acide carboxylique de formule générale III

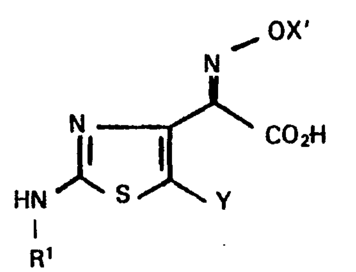

(III)

ou un de ses dérivés activés, dans laquelle formule Y a la signification indiquée ci-dessus, $R^1$ représente l'hydrogène ou un groupe amino-protecteur connu dans la chimie des peptides et X' représente X, un groupe facilement éliminable ou un groupe

$$-CH_2CO_2R^3$$

dans lequel $R^3$ a la signification d'un groupe éliminable dans des conditions modérées, pour obtenir un composé de formule générale IV

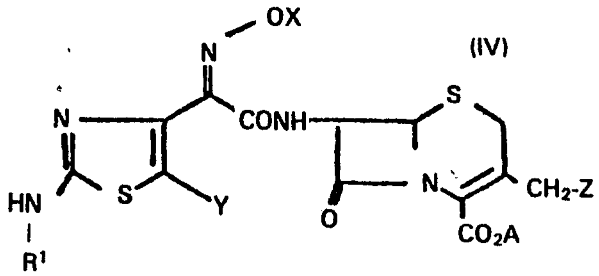

(IV)

dans laquelle $R^1$, X', Y, Z' et A ont les significations ci-dessus, on élimine le groupe $R^1$ ayant la signification d'un groupe protecteur, on convertit le groupe X' en X, dans la mesure où il n'a pas la signification de X, et on convertit Z' en Z, dans la mesure où il n'a pas la signification de Z, l'ordre des opérations d'élimination du groupe $R^1$ et de conversion de X' en X et de Z' en Z étant interchangeable à volonté.

3. Composition pharmaceutique caractérisée par une teneur en composés de formule générale I.

4. Procédé de préparation de compositions pharmaceutiques, caractérisé en ce qu'on met un composé de formule générale I, sous une forme d'administration pharmaceutiquement appropriée, éventuellement avec des véhicules ou diluants pharmaceutiquement usuels.

**Revendication pour l'Etat contractant:**
AT

Procédé de préparation de dérivés de céphalosporine de formule générale I

(I)

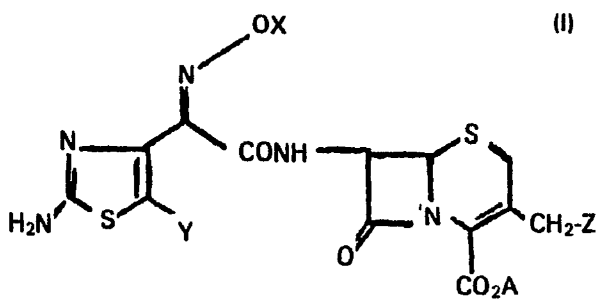

dans laquelle, X représente l'hydrogène, un groupe alcoyle en $C_1$-$C_4$, qui peut être substitué par un halogène, un groupe hydroxy, amino, monoalcoyl ($C_1$-$C_4$)-amino ou dialcoyl($C_1$-$C_4$)-amino, un groupe carboxyméthyle qui peut également être présent sous la forme de ses sels physiologiquement acceptables, un groupe alcoxycarbonylméthyle ayant de 1 à 4 atomes de carbone dans la partie alcoxy, un groupe aminocarbonylméthyle ou cyanométhyle, dans lesquels les groupes carboxy, alcoxycarbonyle, aminocarbonyle ou cyanométhyle peuvent être une ou deux fois substitués par un groupe alcoyle en $C_1$-$C_4$ sur le groupe méthylène, et deux substituants alcoyle pouvant également être fermés en un noyau carbocyclique ayant de 3 à 6 chaînons, Y représente un halogène, Z représente un groupe azide, pyridinium, 4-aminocarbonylpyridinium ou un groupe S-Het, dans lequel Het représente un noyau hétérocyclique à 5 chaînons, qui en dehors d'un atome d'azote, peut encore contenir d'autres hétéroatomes tels que l'azote, le soufre, ou l'oxygène, ou un noyau hétérocyclique à 6 chaînons ayant de 1 à 3 atomes d'azote, ces noyaux à 5 et 6 chaînons pouvant être encore également totalement ou partiellement hydrogénés et encore une ou deux fois substitués par un groupe alcoyle en $C_1$-$C_4$, trifluorométhyle, carboxy, carboxyméthyle, alcoxy($C_1$-$C_4$)-carbonylméthyle, hydroxy, hydroxyméthyle, oxo, amino, alcoyl($C_1$-$C_4$)-amino, dialcoyl($C_1$-$C_4$)-amino, acyl($C_1$-$C_4$)-amino, thiényle ou pyridyle, et A représente l'hydrogène, un groupe ester facilement éliminable ou un cation physiologiquement acceptable, lequel procédé est caractérisé en ce qu'on fait réagir des lactames de formule générale II

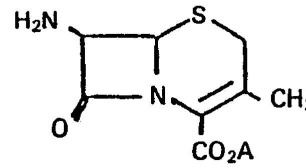

(II)

dans laquelle A a la signification indiquée ci-dessus et Z' représente Z, un groupe acyloxy ayant de 1 à 4 atomes de carbone dans la partie acyle ou un halogène, avec un acide carboxylique de formule générale III

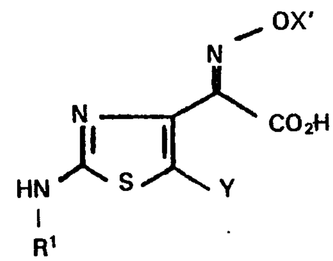

(III)

ou un de ses dérivés activés, dans laquelle formule Y a la signification indiquée ci-dessus, $R^1$ représente l'hydrogène ou un groupe amino-protecteur connu dans la chimie des peptides et X' représente X, un groupe facilement éliminable ou un groupe

$$—CH_2CO_2R^3$$

dans lequel $R^3$ a la signification d'un groupe éliminable dans des conditions modérées, pour obtenir un composé de formule générale IV

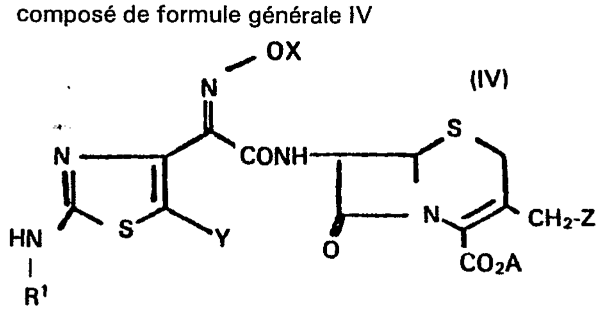

(IV)

dans laquelle $R^1$, X', Y, Z' et A ont les significations ci-dessus, on élimine le groupe $R^1$ ayant la signification d'un groupe protecteur, on convertit le groupe X' en X, dans la mesure où il n'a pas la signification de X, et on convertit le groupe Z' en Z, dans la mesure où il n'a pas la signification de Z, l'ordre des opérations d'élimination de $R^1$ et de conversion de X' et Z' en X et Z étant interchangeable à volonté.

**Claims for the Contracting States:**
BE, CH, DE, FR, GB, IT, LI, NL, SE

1. Cephalosporin derivatives of the general formula I

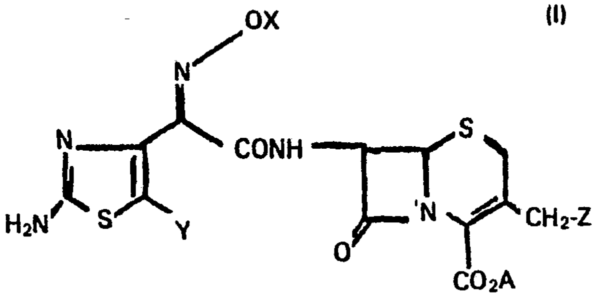

(I)

in which X represents hydrogen, $(C_1$-$C_4)$-alkyl which may be substituted by halogen, by hydroxy, by amino, by mono-$(C_1$-$C_4)$-alkylamino, or by di-$(C_1$-$C_4)$-alkylamino, carboxymethyl which can also be present in the form of its physiologically acceptable salts, alkoxycarbonylmethyl having 1 to 4 C atoms in the alkoxy part, aminocarbonylmethyl or cyanomethyl, wherein carboxymethyl, alkoxycarbonylmethyl, aminocarbonyl or cyanomethyl can be substituted once or twice in the methylene group by $(C_1$-$C_4)$-alkyl and wherein two alkyl substituents can also be closed to form a 3- to 6-membered carbocyclic ring, Y represents halogen, Z represents azide, pyridinium, 4-aminocarbonyl pyridinium or -S-Het, Het denoting a 5-membered heterocyclic ring, which can contain besides a nitrogen atom as further hetero atoms also nitrogen, sulfur or oxygen or a 6-membered heterocyclic ring containing 1 to 3 nitrogen atoms, wherein these 5- and 6-membered rings can also be totally or partially hydrogenated and which can also be monosubstituted or disubstituted by $(C_1$-$C_4)$-alkyl, trifluoromethyl, carboxyl, carboxy methyl, $(C_1$-$C_4)$-alkoxy-carbonylmethyl, hydroxy, hydroxymethyl, oxo, amino, mono-$(C_1$-$C_4)$-alkylamino, di-$(C_1$-$C_4)$-alkylamino, $(C_1$-$C_4)$-alkanoylamino, thienyl or pyridyl and A represents hydrogen, an ester radical which can easily be cleaved or a physiologically acceptable cation.

2. A process for the preparation of cephalosporin derivatives of the general formula I which comprises reacting lactams of the general formula II

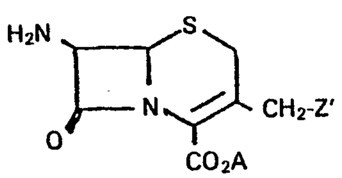

(II)

in which A has the meaning given above and Z' represents Z, acyloxy having 1-4 C atoms in the acyl part or halogen, with a carboxylic acid of the general formula III

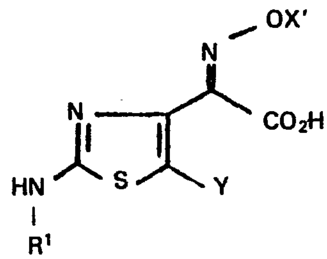

(III)

or with an activated derivative thereof, in which formula Y has the abovementioned meaning, $R^1$ represents hydrogen or an amino protective group known from peptide chemistry and X' represents X, a group which can easily be cleaved or a group

$$—CH_2CO_2R^3$$

in which $R^3$ denotes a radical which can be cleaved under mild conditions, to give a compound of the general formula IV

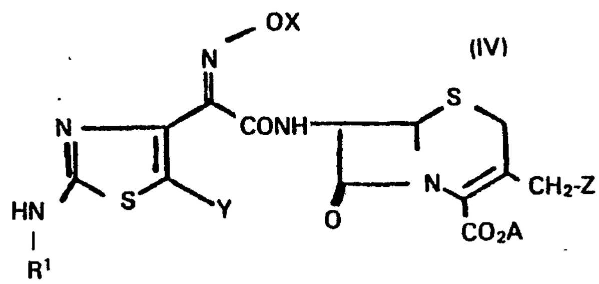

(IV)

in which $R^1$, X', Y, Z' and A have the abovementioned meanings, cleaving the radical $R^1$ when it denotes a protective group, converting the radical X' to X, in the case where it does not denote X, and converting the radical Z' to Z, in the case where it does not denote Z, the order in which the cleavage of $R^1$ and the conversion of X' and Z' to X and Z take place being interchangeable as desired.

3. A pharmaceutical preparation which contains an amount of compounds of the general formula I.

4. A process for the preparation of pharmaceutical preparations, which comprises converting a compound of the general formula I to a pharmaceutically suitable form of administration, if appropriate with pharmaceutically customary carriers or diluents.

**Claim for the Contracting State:**
AT

Process for the preparation of cephalosporin derivatives of the general formula I

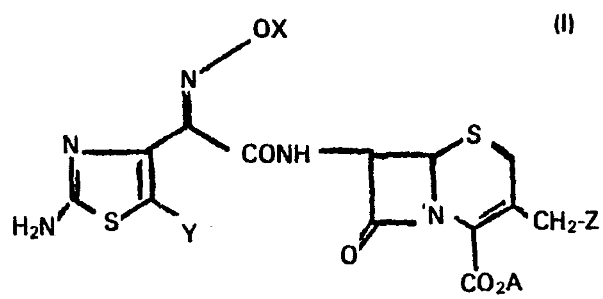

(I)

in which X represents hydrogen, ($C_1$-$C_4$)-alkyl which may be substituted by halogen, by hydroxy, by amino, by mono-($C_1$-$C_4$)-alkylamino, or by di-($C_1$-$C_4$)-alkylamino, carboxymethyl which can also be present in the form of its physiologically acceptable salts, alkoxycarbonylmethyl having 1 to 4 C atoms in the alkoxy part, aminocarbonylmethyl or cyanomethyl, wherein carboxymethyl, alkoxycarbonylmethyl, aminocarbonyl or cyanomethyl can be substituted once or twice in the methylene group by ($C_1$-$C_4$)-alkyl and wherein two alkyl substituents can also be closed to form a 3- to 6-membered carbocyclic ring, Y represents halogen, Z represents azide, pyridinium, 4-aminocarbonyl pyridinium or -S-Het, Het denoting a 5-membered heterocyclic ring, which can contain besides a nitrogen atom as further hetero atoms also nitrogen, sulfur or oxygen or a 6-membered heterocyclic ring containing 1 to 3 nitrogen atoms, wherein these 5- and 6-membered rings can also be totally or partially hydrogenated and which can also be monosubstituted or disubstituted by ($C_1$-$C_4$)-alkyl, trifluoromethyl, carboxyl, carboxy methyl, ($C_1$-$C_4$)-alkoxy-carbonylmethyl, hydroxy, hydroxymethyl, oxo, amino, mono-($C_1$-$C_4$)-alkylamino, di-($C_1$-$C_4$)-alkylamino, ($C_1$-$C_4$)-alkanoylamino, thienyl or pyridyl and A represents hydrogen, an ester radical which can easily be cleaved or a physiologically acceptable cation, which comprises reacting lactams of the general formula II

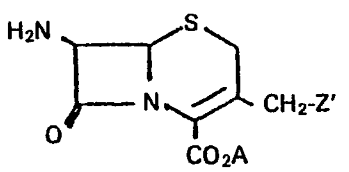

(II)

in which A has the meaning given above and Z' represents Z, acyloxy having 1-4 C atoms in the acyl part or halogen, with a carboxylic acid of the general formula III

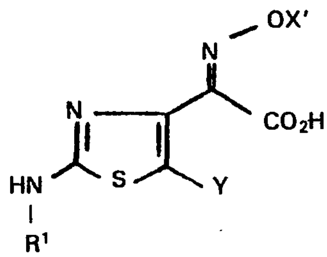

(III)

or with an activated derivative thereof, in which formula Y has the abovementioned meaning, $R^1$ represents hydrogen or an amino protective group known from peptide chemistry and X' represents X, a group which can easily be cleaved or a group

$$—CH_2CO_2R^3$$

in which $R^3$ denotes a radical which can be cleaved under mild conditions, to give a compound of the general formula IV

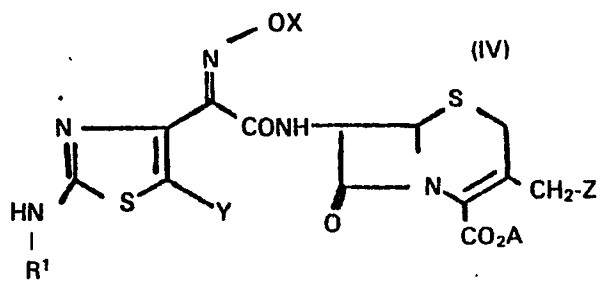

(IV)

in which $R^1$, X', Y, Z' and A have the abovementioned meanings, cleaving the radical $R^1$ when it denotes a protective group, converting the radical X' to X, in the case where it does not denote X, and converting the radical Z' to Z, in the case where it does not denote Z, the order in which the cleavage of $R^1$ and the conversion of X' and Z' to X and Z take place being interchangeable as desired.